(19) Europäisches Patentamt / European Patent Office / Office européen des brevets

(11) **EP 3 064 592 A1**

(12) **EUROPEAN PATENT APPLICATION**

(43) Date of publication:
07.09.2016 Bulletin 2016/36

(51) Int Cl.:
*C12Q 1/68* *(2006.01)*

(21) Application number: **15000655.9**

(22) Date of filing: **06.03.2015**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**MA**<br><br>(71) Applicant: **König, Brigitte**<br>**58119 Hagen (DE)** | (72) Inventor: **König, Brigitte**<br>**58119 Hagen (DE)**<br><br>(74) Representative: **Castell, Klaus**<br>**Patentanwaltskanzlei**<br>**Liermann - Castell**<br>**Willi-Bleicher-Strasse 7**<br>**52353 Düren (DE)** |

(54) **Methods for the qualitative and quantitative detection of microbes in a sample**

(57) The present invention relates to methods for the qualitative and absolute quantitative detection of microbes in a sample. The present invention quantifies microbes according to their original abundance in the sample. The present invention relates to methods for the simultaneous quantitative analysis of structural diversity of different communities (such as bacterial, fungal, archeal, parasites, viruses etc.). In this regard, the method can include providing a plurality of microbial profiles, wherein each microbial profile is established from a sample of microorganisms obtained from an individual subject. The method can also include identifying one or more consensus profiles from among the plurality of microbial profiles. The present invention relates to methods for the simultaneous qualitative and/or absolute quantitative analysis of functional genes. These methods can be used more specifically to characterize normal, disturbed and pathological microflora in vaginal samples from non-pregnant and pregnant women and to determine preterm risk. These methods can be used to diagnose e. g. bacterial vaginosis among others infection related pathologies and to be used in prevention preterm delivery. Moreover, the present invention also relates to distinctive kits for the qualitative and/or quantitative detection of microbes/microbial profiles adapted to specific areas of medicine (e.g. vaginosis), food and pharmaceutical production, environmental and ecology studies, forensic matters and life sciences including a specified kit for vaginal samples.

EP 3 064 592 A1

**Description**

CROSS-REFERENCE TO RELATED APPLICATIONS

This application claims reference to Patent Application No. EP13173893, filed June 23, 2013, the entirety of which is incorporated by reference herein.

BIBLIOGRAPHY

Several documents are cited throughout the text of this specification. The disclosure content of these documents, including any manufacturer's specification, is incorporated by reference

ABSTRACT

The present invention relates to methods for the qualitative and absolute quantitative detection of microbes in a sample. The present invention quantifies microbes according to their original abundance in the sample. The present invention relates to methods for the simultaneous quantitative analysis of structural diversity of different communities (such as bacterial, fungal, archeal, parasites, viruses etc.). In this regard, the method can include providing a plurality of microbial profiles, wherein each microbial profile is established from a sample of microorganisms obtained from an individual subject. The method can also include identifying one or more consensus profiles from among the plurality of microbial profiles. The present invention relates to methods for the simultaneous qualitative and/or absolute quantitative analysis of functional genes. These methods can be used more specifically to characterize normal, disturbed and pathological microflora in vaginal samples from non-pregnant and pregnant women and to determine preterm risk. These methods can be used to diagnose e. g. bacterial vaginosis among others infection related pathologies and to be used in prevention preterm delivery. Moreover, the present invention also relates to distinctive kits for the qualitative and/or quantitative detection of microbes/microbial profiles adapted to specific areas of medicine (e.g. vaginosis), food and pharmaceutical production, environmental and ecology studies, forensic matters and life sciences including a specified kit for vaginal samples..

FIELD OF THE INVENTION

This disclosure relates to the field of microbial ecology. The present disclosure relates to methods for the qualitative and quantitative detection of microbes in a sample. Additionally, the disclosure relates to methods for identifying and categorizing populations of microbiota quantitatively. Moreover, the present disclosure also relates to a kit for the qualitative and/or quantitative detection of microbes/microbial communities.

DESCRIPTION OF THE RELATED ART

Many industries, including pharmaceuticals and food processing industry, need to detect low levels of biological material in large volumes of liquid. In industry, areas often under surveillance by Quality Assurance personnel include the control of bioburden in incoming raw materials; monitoring of microbial population in the production environment; in-process controls, especially alter storage; and final product testing. Often product is manufactured and stored whilst analysis for microbial content takes place. If contamination is detected, the product may need to be destroyed, and the production line shut down until the source of contamination is found. Often the time taken for microbial analysis is the rate-limiting factor in bringing the plant on stream. This can lead to substantial costs in wasted production or raw materials.
Similarly in health care, the specimen are analyzed in order to characterize the presence of a microorganism or parasite whilst the negative effect of the infection may progress and a severe disease may be established. Again, the time taken for microbial analysis may be a rate-limiting factor, often deciding about the fate of the patient. Moreover, diagnosis in health care also needs to be able to discriminate between closely related organisms present in the specimen which can be a laborious and sometimes impossible for conventional analysis techniques. Moreover, in health care the analysis and monitoring of the balance/dysbalance between different microorganisms or groups of microorganisms or different kingdoms of microorganisms (e.g. bacteria and fungi) is becoming more and more important. Moreover, recent research indicates that in many cases the sole detection of a microorganism without the analysis of functionality is not sufficient.
Bacterial vaginosis (BV) is the most prevalent cause of vaginitis among women of childbearing age (Holmes et al., Sexually Transmitted Diseases (1999)). The prevalence of BV in women varies depending on the population studied, but ranges from 4% to >50%. The occurrence of BV is associated with an increased risk of acquiring sexually transmitted diseases (STDs) including HIV (Martin et al., J Infect Dis 178:1053-1059 (1998); Schmid et al., Sex Transm Infect 76:3-4 (2000); Taha et al., AIDS 12:1699-1706 (1998); Sobel, Annu Rev Med 51:349-356 (2000); Schwebke, Curr Infect Dis Rep 2:14-17 (2000); Gupta et al., J Infect Dis 178:446-450 (1998); Hawes et al., J Infect Dis 174:1058-1063 (1996)), pelvic inflammatory disease, and various postoperative infections (Sobel, Annu Rev Med 51:349-356 (2000); Pybus & Onderdonk, Microbes Infect 1:285-292 (1999), and references therein) as well as delivery of spontaneous abortion (Leitich et al., Am J Obstet Gynecol 189:139-147 (2003); Ralph et al., BMJ 319:220-223 (1999)), preterm infertility (Sweet, Infect Dis Obstet Gynecol 8:184-190 (2000)), preterm delivery of low and very low birth weight infants (Hillier et al., N Engl J Med 333:1737-1742 (1995). Preterm birth before 37 weeks' gestation is the greatest cause of perinatal mortality and morbidity in the developed world, with the worst outcomes seen in babies born before 30 weeks. It is associated with 50% of childhood neurological disability, including cerebral palsy and blindness. In addition the social and financial burden is enormous.

Classical methods of microbe detection and characterization, whilst considered reliable and accepted, are generally time consuming, cost intensive and less sensitive than desired. Moreover, it was shown that more than 85% of the organisms existing in nature appear to be refractory to culture-dependent methods (Amann, et al., Microbiol. Rev. 1995, 59, 143-169; Barns, et ah, Nati. Acad. Sci. USA. 1994, 91, 1609-1613; Giovannoni, et al." Nature 1990, 345, 60-63; Torsvik, et ah, Appl. Environ. Microbiol. 1990, 56, 782-787; Ward, et al, *Nature.* 1990, 345,6365). As a consequence, the natural community structure can be altered by the application of selective conditions during culture which do not relate to environmental parameters. Therefore, a new community structure evolves, which may not accurately reflect the original structure. Biochemical detection approaches often require a pure culture. Therefore, this method shares the described problems.

From the microbiological point of view, BV is a polymicrobial syndrome characterized by disturbance of the normal vaginal microbiota and appears to be the result of an aetiologically unknown process in which the physiological microbial flora is replaced by a still ill-defined set of mostly anaerobic bacteria. Based on conventional culture identification, an association has been found between BV and the detection of Gardnerella vaginalis, Mobiluncus mulieris and anaerobic Gram-negative bacteria, especially Prevotella spp.. Recent findings, mostly based on molecular methods, have emphasized the association between Atopobium vaginae and BV (Ferris et al., 2004). However, the alleged pivotal role of these bacteria has been questioned, as they were also found in the vaginal fluid of healthy women. Therefore, recent research has focused on the development of culture independent methods for in-depth analysis of vaginal microbiota. Detailed information on the complex microbial communities in BV as well as under normal conditions has been obtained by cloning and sequencing of 16S rRNA gene libraries (Verhelst et al., 2004; Zhou et al., 2004; Hyman et al., 2005; Fredricks et al., 2005). It became aware that in addition to Atopobium vaginae, relatively unknown bacterial species such as 'Leptotrichia amnionii' (Shukla et al., 2002), Eggerthella sp., Sneathia sanguinegens and Megasphaera sp. have been implicated in the pathogenesis of BV. Curiously, up to 50% of women diagnosed with BV may not exhibit all or any of the classic symptoms (Sweet, Infect. Dis. Obstet. Gynecol. 8:184-190 (2000); Schmid, Int. J. Gynaecol. Obstet. 67 Suppl. 1:S17-S20 (1999); Schwebke, Int. J. Gynaecol. Obstet. 67 Suppl. 1:S21-S23 (1999)). Such asymptomatic women are diagnosed as having BV due to the absence of numerically abundant populations with cellular morphologies that resemble those of lactobacilli. The equating of absence of lactobacilli with the occurrence of BV, has gained wide acceptance despite the fact that numerous studies have shown that a significant fraction of women without BV symptoms lack appreciable numbers of lactobacilli.

Many investigators have studied the species composition of vaginal flora and paid heed to the species of *Lactobacillus* present. It is widely believed that the principal *Lactobacillus* species in the vagina of healthy women are *Lactobacillus crispatus, Lactobacillus jensenii,* and *Lactobacillus gasseri* (Antonio et al., J. Infect. Dis. 180:1950-1956 (1999); Pavlova et al., J. Appl. Microbiol. 92:451-459 (2002)). There is, however, disagreement in the literature and various other species have been reported as members of normal vaginal flora. For example, Reid et al. (FEMS. Immunol. Med. Microbiol. 15:23-26 (1996)) sampled 100 healthy premenopausal women and cultivated the dominant aerobic or microaerophilic isolates of *Lactobacillus* from vaginal swab samples. Eight species were detected, the most common species being *L. jensenii.* The uncertainty regarding the actual species of *Lactobacillus* in the human vagina can in part be attributed to the difficulties of classifying lactobacilli on the basis of phenotypic criteria and the historical confusion surrounding the taxonomy of *Lactobacillus.* While investigators have focused attention on the role and importance of lactobacilli as members of the vaginal flora, the fact that between 10 and 42% of women lack appreciable numbers of lactobacilli (Eschenbach et al., Clin. Infect. Dis. 30:901-907 (2000); Hillier, AIDS Res. Hum. Retroviruses 14 Suppl 1: S17-S21 (1998); Larsen Monif, Clin. Infect. Dis. 32:e69-e77 (2001); Marrazzo et al., J. Infect. Dis. 185:1307-1313 (2002); Redondo-Lopez et al., Rev. Infect. Dis. 12:856-872 (1990)) has been nearly overlooked. Thus, up to date it is not known which variations in vaginal flora belong to a healthy / intermediate or ill status. Moreover, it is not known which vaginal flora is predictive for the development of vaginosis as well as for preterm delivery.

Diagnosis of BV is based on the presence of clinical signs as well as microbiological findings. Among the many laboratory methods used for the diagnosis of BV, the Gram-stain criteria as defined by Nugent et al. (1991) are currently regarded as the standard procedure. Although this method has been repeatedly refined (Pereira et al., 2005; Verhelst et al., 2005), morphological assessment alone may not reflect the microbiological variations among BV individuals and should be extended by molecular methods (Fredricks & Marrazzo, 2005). Prior efforts to characterize the vaginal flora have largely employed methods that are commonly used in clinical microbiology laboratories (Redondo-Lopez et al., Rev. Infect. Dis. 12:856-872 (1990), and references therein). These methods are inherently limited because they require cultivation of organisms on selective and nonselective media in the laboratory, after which they are classified into broad taxonomic groups based on phenotypic characters and microscopy. Slow growing, strictly anaerobic, or fastidious organisms may not be recovered by these methods. Others may have failed to grow because investigators are unaware of their inability to grow on selective media. Finally, the coarse classification methods used do not distinguish ecotypically distinct populations in samples. Traditional culture-dependent methods are tedious and labor intensive, and their use for the analysis of large numbers of samples is costly, permitting analysis of only small numbers of samples per study. Inventories of resident human bacterial flora done using cultivation-independent approaches based on analyses of 16S rRNA gene sequences have revealed a large degree of previously uncharacterized diversity even within well-studied and familiar microbial environments such as the human gingival crevice (Kroes et al., Microbiol. 96:14547-14552 (1999); Paster et al., J. Bacteriol. 183:3770-3783 (2001)), intestines (Favier et al., App. Environ. Microbiol. 68:219-226 (2002); Zoetendal et al., J. Nutr. 134:465-472 (2004)), inner ear (Frank et al., J. Clin. Microbiol. 41:295-303 (2003)), tongue (Kazor et al., J. Clin. Microbiol. 41:558-563 (2003)), and the esophagus (Pei et al., Proc. Natl. Acad. Sci. USA 101:4250-4255 (2004)).

Recent prior art provides a detection method based the determination of genus correlated restriction fragment length polymorphisms (RFLPs) of the rDNA (Liu, et al., Appl. Environ, Microbiol. 1997, 63, 4516-4522). The combination of RFLP with PCR amplification by using fluorescence labeled universal primers, restriction enzymes with 4-bp recognition sites and high-resolution polyacrylamide gels in laser-scanning gel sequencers allows a qualitative and semi-quantitative analysis of the community-specific pattern. However, the method suffers a number of serious limitations that prevent a diagnostic application. Specifically, none of the analytical primers used (Liu at ah, 1997) allowed a simultaneous detection of eukaryal, bacterial, parasitic, and archael nucleic acid sequences. Additionally, the apparent abundance of different populations tends to be skewed in presently available diagnostic methods, thus preventing a balanced representation of the microbes present in the sample. Recent inventions using T-RFLP are not able to give quantitative results either on single, multiple microorganisms or on microbial profiles. Finally, the gel sequencers, exclusively used at present, are expensive, need skewed personal in molecular biology, are difficult to automate for small sample numbers and therefore are very labour and cost intensive.

Therefore, the technical problem underlying the present Invention was to provide means and methods that allow fast and efficient qualitative detection, an absolute enumeration and a real absolute relationship to each other including their functionality.

## DETAILED DESCRIPTION

The solution to the above technical problem is achieved by providing the embodiments characterized in the claims.

In order to facilitate review of the various embodiments of this disclosure, the following explanations of specific terms are provided:

The term "microbe" refers to an organism that is too small to be visible with the naked eye, and is used synonymously with the term "microorganism." Microorganisms include bacteria, (Archaea, Eubacteria), yeast, fungi, parasites and for the purposes of this disclosure, shall be understood to include viruses. Parasites include, but are not limited to, multi-cellular organisms such as, e.g. Cestodes, Tremades or Nematodes. A microorganism can be formed by a single cell er by a small number of cells. Bacteria and Protozoa are examples of microorganisms that comprise a single cell. Some microorganism like, e.g., fungi, cycle through two or more developmental stages ranging from a multi-cellular organism to a single cell, both of which are encompassed by the term "microbe" and the term "microorganism". However, both terrns also refer to the spores of fungi and similar devices of reproduction derived from other organisms, provided they contain the genetic information of the microbe. Viruses are further examples of microorganisms and include enveloped and non-enveloped viruses as well as bacteriophages. However, the term viruses also includes infectious nucleic acid molecules, such as those of viroids, which are not associated with a coat but are capable of replicating themselves. The term "species of microorganism" is used herein to refer to a taxonomically and/or genetically distinct group of microorganism. The term "predominant species" (for example, predominant species of microorganism) refers to one or more species that is/are numerically more frequent than other species in a mixed sample or population. For example, a predominant species may be the most numerically frequent species in a mixed sample or population, or a predominant species may be one of several numerically frequent species present in a mixed sample or population. In an embodiment, a predominant species is at least 10% of the mixed sample or population. For example, a predominant species can be at least 20%, or at least 30%, frequently greater than about 40%, or greater than 50% of the mixed population. In some cases, the predominant species is often than about 60%, sometimes greater than about 70%, and can be greater than 80% or even 90% of the mixed sample or population. In another embodiment, a predominant species is at least 2x as prevalent in the mixed sample as another species of microorganism. Alternatively, the predominant species is at least 3x as frequent in the mixed sample as other organisms. In some cases, the predominant species is at least 4x, or at least about $5\times$, or even as much as $10\times$ as frequent in the mixed sample or population than another species of microorganism.

The term "microbiota" refers to an assemblage of microorganism localized to a distinct environment. For example, "vaginal microbiota" are an assemblage of one or more species of microorganisms that are localized to, or found in, a vagina. "Normal vaginal microbiota" are a population of microorganisms that localize to the vagina in a normal, that is, a non-pathological or non-pathogenic, state. For example, a sample of normal vaginal microbiota is obtained from a woman without a vaginal pathology, that is, from a woman with no sign or symptom corresponding to or resulting from a pathology of the vagina. Normal vaginal microbiota can be obtained from a woman with a pathology of an organ or tissue other than the vagina. In a medical context, the term "microflora" is often used synonymously with the term "microbiota."

The term "microbial community" refers to one or more microbial populations found together in a shared environment. For example, a shared environment can be a defined site or location on or in a subject (e.g., a host), or can be an environmental site or location not associated with a subject. Thus, a shared environment can be a specific organ or tissue within the body of a subject, such as the skin, the oral cavity, the gingival crevice, the esophagus, the ear, the small intestine, the large intestine, the rectum, the vagina, etc. Alternatively, a shared environment can be a site or location, such as soil, water, or another environmental source not pertaining to a particular subject (such as a human subject). For purposes of clarity, samples obtained from an environment on or in (pertaining to a) subject will be so designated, for example, a sample obtained from a human subject, a sample obtained from an animal subject, a sample obtained from a plant subject. In contrast, a sample obtained from a source other than a subject will be referred to as an "environmental sample."

Individual species of microorganisms obtained from a subject, such as a human, animal or plant subject, may exist in various relationships with respect to the subject (or host). For example, the microorganism can be a "symbiotic microorganism" that exists in a relationship with its host that provides a benefit to both the microorganism and the host, that is, a mutually beneficial relationship. The microorganism also can be a "commensal microorganism" that exists in a relationship that is beneficial to the microorganism and neither benefits nor harms the host. Alternatively, the microorganism can be a "parasitic microorganism" that derives benefit from its host at the expense of or detriment to the host. Additionally, a microorganism can be a "pathogenic microorganism" that causes or is capable of causing a disease state or condition in the host.

A "microbial profile" is a set of the species and/or strains of microorganisms present in a sample of microorganisms. To the extent that a sample of microorganisms is obtained from, and corresponds to the species found in, a shared environment, the microbial profile details the species present in a microbial community. The term "consensus profile" is used herein to refer to the species common to multiple samples with similar microbial profiles. That is, a consensus profile includes the species of microorganisms that are common to each of multiple samples, which may or may not have additional unshared species.

One embodiment is a method for the qualitative detection of microbes in a sample comprising: (a) lysis of microbes under conditions that give access to the nucleic acids from all mentioned microorganisms (bacteria, archae, parasites, eukaryotes, viruses) equally ; (b) amplification of at least a portion of the microbial nucleic acid with at least one primer pair consisting of one labeled and one unlabeled oligonucleotide or of at least two differently labelled oligonucleatides; (c) sequence-specific fragmentation of the amplified DNA; (d) determination of the length of the DNA fragments into which the labelled oligonucleotide is incorporated; (e) comparison of the length of the DNA fragments of step (c) with DNA fragments predicted from the DNA sequence of microbes, wherein the nucleotide sequence is taken from a database and the DNA sequence is fragmented according to the principles underlying the sequence specific fragmentation of step (c); and (f) detecting whether a microbe is present in said sample; whereby the identity in length of the DNA fragment referred to in step (d) and of a DNA fragment predicted from the DNA sequence of the microbe referred to in step (e) is indicative of the presence of said microbe in said sample.

Unless otherwise explained, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press (1994)(ISBN 0-19-854287-9); Kendrew et al., (eds.), The Encyclopedia of Molecular Biology, published by Blackwell Science Ltd. (1994)(ISBN 0-632-02182-9); and Robert A. Meyers (ed.), Molecular Biology and Biotechnology: a Comprehensive Desk Reference, published by VCH Publishers, Inc. (1995)(ISBN 1-56081-569-8).

The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described below. The term "comprises" means "includes."The abbreviation, "e.g." is derived from the Latin exempli gratia, and is used herein to indicate a non-limiting example. Thus, the abbreviation "e.g." is synonymous with the term "for example.

The term "lysis"_refers to the process of generating holes in the membrane, coat or wall of the microbe. To amplify a target nucleic acid sequence in a sample, e.g. by PCR, the sequence (-flust be accessible to the components of the amplification system. In many cases conditions of limited lysis may be sufficient for nucleic acid amplification. in this case, the holes need to be !arge enough to allow access of small macromolecules such as, e.g. replication enzymes. However, accessibility of microbial nucleic acid molecules may also be accomplished by complete lysis of the microbe and/or by isolating the nucleic acids from the sample. A variety of techniques for extracting nucleic acids from biological samples are known in the art. For example, see those described in Rotbart et al., 1989, in PCR Technology (Erle ed., Stockton Press, New York) and Han et af. 1987, Biochemistry 26:1617-1625. if the sample is fairly readily disruptable, the nucleic acid need not be purified prior to amplification by the PCR technique, i.e., if the sample is comprised of cells, e.g. peripheral blood lymphocytes or monocytes, lysis and dispersion of the intracellular components may be accomplished merely by suspending the cells in hypotonic Buffer. Suitable methods will vary depending on the type of microorganism and are well known to the person skilled in the art (see e.g. Sambrook et al.,). The person skilled in the art also knows how to adopt and optimise these methods in order to achieve optimal results for the specific microbe.

The terms "nucleic acicl", "nucleic acid mofecufe" or "polynucleotide" refer to DNA or RNA mofecuies which may be single stranded or double stranded or partiaily double stranded. Double stranded mofecules include DNA/DNA double strands as well as RNA/RNA double strands or DNA/RNA hybrid double strands. in some cases the nucieic acid molecule may be covalentfy closed, generating a circufar molecule. The ciosure may also be mediated by proteins that bind to both ends of a linear nucleic acid mofecufe. Furthermore, as used herein, nucleic acid mofecules can also be chemically synthesized or can contain modified nucleotides or nucleotide derivatives. A number of modifications have been described that alter the chemistry of the phosphodiester backbone, sugars or heterocyclic bases. Among useful changes in the backbone chemistry are phosphorothioates; phosphorodithioates, where bothof the non-bridging oxygens are substituted with suffur; phosphoroamidites; alkyl-phosphotriesters and boranophosphates. Achiraf phosphate derivatives include 3'-O-5'-S-phosphorothioate, 3'-S-5'-0-phosphorothioate, 3'CH2-5$^1$-0-phosphonate and 3'-NH-5'-0-bhosphoroamidate.

Peptide nucleic acids replace the entire phosphodiester backbone with a peptide linkage. Sugar modifications are also used to enhance stability and affinity. The aanomer of deoxyribose may be used, where the base is inverted with respect to the natural b-anomer. The 2-OH of the ribose sugar may be altered to form 2$^1$-0-methyl or 2'-0-allyi sugars, which provides resistance to degradation without comprising affinity. Modification of the heterocyclic bases must maintain proper base pairing. Some useful substitutions include deoxyuridine for deoxythymidine; 5-methyl-2'- deoxycytidine and 5-bromo-2'-deoxycytidine for deoxycytidine. 5-propynyl-2'- deoxyuridine and 5-propynyr-2'-deoxycytidine have been shown to increase affinity and biological activity when substituted for deoxythymidine and deoxycytidine, respectively.

The term "primer" refers to an oligonucieotide, whether nature.' or synthetic, capable of acting as a point of initiation of DNA synthesis under conditions in which synthesis of a primer extension product complementary to a nucleic acid strand is induced, i.e., in the presence of Tour different nucleoside triphosphates or analogues thereof and an agent for polymerisation (i.e., DNA polymerase or reverse transcriptase) in an appropriate buffer and at a suitable temperature. A primer is a nucleic acid molecule as described above. Preferably, a primer is a single-stranded oligodeoxyribonucleotide. The appropriate length of a primer depends an the intended use of the primer but typically ranges from 10 to 25 nucleotides. Short primer malewies generally require cooler temperatures to form sufficiently stabie hybrid complexes with the template. A primer need not reflect the exact sequence of the template but must be sufficiently complementary to hybridize with a template.

"Hybridize" refers to the binding of two single stranded nucleic acids via complementary base pairing, i.e. A to T (in RNA: U), G to C. The term "primer pair" refers to two primers that hybridize with the + and - strand, respectively, of a double stranded nucleic acid molecule, and allow the amplification of DNA fragments, as for example in a PCR reaction. A primer can be labelled, if desired, by incorporating a compound detectable byspectroscopic, photochemical, biochemical, immunochemical, or chemical means. For example, useful labeis inclucie fluorescent dyes, electron-dense reagents, biotin, or small peptides for which antisera or monoclonal antibodies are avallabb. A label can also be use⁻cl to "capture" the primer, so as to facilitate a selection of amplified nucleic acid or fragments thereof. Carboxyffuorescein (FAM) and 6-carboxy-X-rhodamine (ROX), are preferred labels. However, other preferred labels include fluorochromes, e.g. fluorescein isothiocyanate (FITC), rhodamine, Texas Red, phycoerythrin, allophycocyanin, 6- carboxyfluorescein (6-FAM), 2',7'-dimethoxy-4,5'-dichloro-6-carboxyfluorescein (JOE), 6-carboxy-24,7',4,7-hexachlorofluorescein (HEX), 5-carboxyfluorescein (5FAM) or N,N,KIV-tetramethyl-6-carboxyrhodamine (TAMRA), radioactive labels, e.g. $^{32}$P, $^{35}$S, $^3$H; etc. The label may also be a two stage system, where the DNA is conjugated to biotin, haptens, etc. having a high affinify binding partner, e.g. avidin, specific antibodies, etc., where the binding partner is conjugated to a detectable label. In the case of amplification the iabel may be conjugated to one or both of the primers.

In the present Invention, the term "amplification" or "amplify" means increase in copy number. The Person skilled in the art know various methods to amplify nucleic acid molecules. Amplification methods include, but are not fimited to, "polymerase chain reaction" (PCR), "ligase chain reaction"(LCR, EPA320308), "cyclic probe reaction" (CPR), "strand displacement amplification" (SDA, Walker et al. 1992, Nucleic Acid Res. (7): 1691-1696), "transcription based amplification systems" (TAS, Kwöh et al. 1989, Proc. Nat. Acad. Sci. USA 86: 1173, Gingeras et al., PCT Application WO 88/10315).

Preferably, amplification of DNA is accomplished by using polymerase chain reaction (PCR). The PCR consists of many repetitions of a cycle which consists of: (a) a denaturation step, which melts both strands of a DNA molecule; (b) an annealing steep, which is aimed at allowing the primers to anneal specifically to the melted strands of the DNA molecule; and (c) an extension step, which incorporates to the primers complementary to those of the strand of DNA to which the primers are annealed. The concentrations of primers, nucleotidetriphosphates, enzyme and buffers used will be apparent from and include the process parameters described in the Examples that follow. However, generally, PCR can be performed for example in a 50. p1 reaction mixture containing 5 $p1$ of 10 x PCR buffer with 1.5mM mM MgCl2, 200 pM of each deoxynucleoside triphosphate, 0.5 $p1$ of each primer (10 pM), 0.5 30 ng of microbial genomic template DNA and 2.5 Units of Taq Palmerase. The primers for the amplification may be [abelied or be uniabelled. DNA amplification can be performed, e.g., with a mode] 2400 thermal cycler (Applied Biosystems, Foster City, CA): 2 min at 94°C, followed by 35 cycles consisting of annealing (30 s at 50°C), extension (1 min at 72°C), denaturation (10 s at 94°C) and a final annealing step at 55°C for 1 min as weil as a final extension step at 72°C for 5 min. However, the person skilled in the art knows how to optimize these conditions for the amplification of specific nucleic acid molecules.

A further method of nucleic acid amplification is the "reverse transcriptase polymerase chain reaction" (RT-PCR). This method is used when the microbial nucleic acid to be amplified consists of RNA. The term "reverse transcriptase" refers to an enzyme that catafyzes the polymerisation of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Eimer. Typically, the genomic RNA/cDNA dupfex template is heat denatured during the first denaturation step after the initial reverse transcription step leaving the DNA strand available as an amplification tempiate. Suitable polymerases for use with a DNA template include, for example, E. coli DNA polymerase 1 or its Kienow fragment, T.sub.4 DNA polymerase, Tth polymerase, and Taq polymerase, a heat-stable DNA polymerase isolated from Thermus aquaticus and developed and manufactured by Hoffmann-La Roche and commercially available from Perkin Eimer. The latter enzyme is widely used in the amplification and sequencing of nucleic acids. The reaction conditions for using Taq polymerase are known in the art and are described, e.g., PCR Technology, Erlich, H, A. 1989. Stockton Press, New York or innis, M. A., D. H. Gelfand, J. J. Sninsky, and T. J. White. 1990. PCR Protocols: A guide to methods and apprications, Academic Press, New York.

High-temperature RT provides greater primer specificity and improved efficiency. Copending U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycies (DNA template). The RT Reaction can be performed, for example, in a 20ul reaction mix containing:4 ul of 5x ANV-RT Buffer 4 ul, 2 ul of Oligo dT(100 ug/mr), 2ul of 10 mM dNTPs ,1 ul total RNA,10 Units of AMV reverse transcriptase and H2O to 20 $\mu$l final volum6. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample.

The term "sequence-specific fragmentation" or "fragmentation" as used herein refers to the fragmentation of nucleic acid molecules at specific nucleotide positions. "Sequence-specific fragmentation" is catalyzed preferably by a restriction enzyme, including restriction endonucleases. However, "sequence-specific fragmentation" may also be performed by any other method known to the Person skilled in the ad provided the cleavage reaction is based an the specificity of the nucleotide sequence of the nucleic acid molecule. The term "principles underlying the sequence specific fragmentation" relates to the factors governing which position of the nucleotide sequence is cleaved. For example, specific restriction enzymes recognize and cleave a specific nucleotide sequence, thus performing the cleavage reaction consistently at the same position of a given nucleic acid molecule. The present Invention only refers to those cleavage reactions that generate consistent cleavages of nucleic acid molecules and depend on the specific succession of nucleotides.

It is well known to the person skilled in the art that restriction enzymes recognize a specific succession of nucleotides within a DNA molecule and catalyze it's cleavage. However, the cleavage position does not need to be identical with the position of the recognition sequence. Hence, the term "restriction enzyme" also refers to restriction enzymes which cleave at a specific position or distance upstream or downstream of a recognition sequence. Preferably the restriction enzyme is a restriction enzyme with a recognition sequence of 4 base pairs or 5 base pairs, including Hha 1, MspI and AciI. A fragmentation reaction catalyzed by a restriction enzyme contains at least one restriction enzyme in a concentration sufficient to cleave the entire amount of double stranded DNA present in the reaction mixture. Suitable reaction conditions vary depending on the specific enzyme and are well known to the person skilled in the art (see e.g. Sambrook et al.,). The person skilled in the art also knows how to adopt these methods in order to achieve optimal results.

The term "determination of fragment length" refers to a method that allows to determine the precise number of nucleotides present in the fragmented nucleic acid molecule. Preferably the method allows the length determination of at least one fragment of the amplified nucleic acid molecule. Also preferred is the determination of the fragment length by using DNA electrophoresis. However, also chromatography including high pressure liquid chromatography (HPLC). or mass spectrometry, or a microfluid-chip-based capillary electrophoresis system are further preferred methods for fragment length determination. When using DNA electrophoresis as a means of determining the fragment length, the mixture containing the fragmented nucleic acid molecules is preferably separated on an automated sequencer device, as for example the 3100 Genetic Analyzer automated sequencer. Preferably this device is equipped with a linear polymer matrix, such as the performance optimized polymer type 6 (Applied Biosystems). When determining the fragment length of a nucleic acid molecule, typically, this is accomplished by comparison of the nucleic acid fragment with a labeled internal size standard such as, e.g., the GENESCAN© 500 ROX and by using e.g. the GENESCAN® Analysis software 3.7.

Finally, determination of the microbe corresponding to the detected nucleic acid fragment sizes may be performed, for example, by using the T-RFLP analysis program (TAP) and Ribosomal Database of the Center for Microbial Ecology at Michigan State University (Maiflak BL, Cole JR, Lilburn TG, Parker CT Jr, Saxman PR, Farris RJ, Garrity GM, Olsen GJ, Schmidt TM, Tiedje JM. The RDP-11 (Ribosomal Database Project) Nucleic Acids Res 2001 Jan 1;29(1):173-4) or the data base for.

In a preferred embodiment of the invention the internal nucleic acid quantity standard is added prior to lysis. A known quantity of an internal standard may be added to the sample prior to lysis. If the nucleic acid molecule to be amplified is DNA, than a specific DNA molecule or a mixture of DNA molecules may be added. Alternatively, if the nucleic acid molecule to be amplified is RNA, than it may be advantageous to add a known quantity of a specific RNA molecule er of a mixture of different RNA molecules. However alternatively, the internal DNA standard may be generated in situ by adding a known amount of one or more microbes to the sample prior to lysis, thus generating the DNA or RNA molecule during the amplification step by amplifying specific internal DNA and/Or RNA molecules of the microbe.

In another preferred embodiment of the invention the internal quantity standard is homologous to an amplified microbial DNA, wherein the DNA comprises repetitive sequences and/or is selected from the group consisting of 16S rDNA, 23S rDNA, TUF, HSPs, ITS or others. Additional examples of phylogenetically informative genes suitable as molecular indicators of identity include: rpoB; gyrB; gyrA; tmR-NA; recA; EF-Tu (tuf); groEL (cnp60, hsp60); atpD; ompA gene; gapA; pgi;fusA; ileS; lepA; leuS; pyrG; recG; rp1B. Other genes (for example functional genes encoding related enzymes that perform a defined function) also can be utilized, at least with respect to narrower groups of microorganisms. Examples of such functional genes include the pmoA/amoA genes; the mmoX gene; the nifH gene; the nirS gene; the nirK gene; the norB gene; the mcrA gene; and the rbcL gene. Of course, one of ordinary skill will appreciate that any polymorphic gene or gene family can be utilized as a molecular indicator of identity. However, the nucleic acid molecule can also be amplified from the transcripts of the aforementioned genes. The term "homologous" refers to a first nucleic acid molecule which has at least 80% identity, preferably at least 90% identity and more preferably 95% identity to a second nucleic acid molecule. As a practical matter, whether any particular nucleic acid molecule is at least 80%, 90% or 95% identical to a second nucleic acid molecule or fragments thereof, can be determined conventionally using known computer programs such as the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman to find the best segment of homology between two sequences (Advances in Applied Mathematics 2:482-489 (1981)). When using Bestfit or any other sequence alignment program to determine whether a particular sequence is, for instance, 95% identical to a reference sequenc, the parameters are set, of course, such that the percentage of identity is calculated over the full length of the reference nucleotide sequence and that gaps in homology of up to 5% of the total number of nucleotides in the reference sequence are allowed. The identity between a first sequence and a second sequence, also referred to as a global sequence alignment, is determined using the FASTDB computer program based on the algorithm of Brutlag and colleagues (Camp. App. Biosci. 6:237-245 (1990)). In a sequence alignment the query and subject sequences are both DNA sequences. An RNA sequence can be compared by converting U's to T's. The result of said global sequence alignment is in percent Identity. Preferred parameters used in a FASTDB alignment of DNA sequences to calculate percent Identity are: Matrix.Unitary, Mismatch Penalty=1, Joining Penalty.30, Randomization Group Length=0, Cutoff Score=1, Gap Penalty=5, Gap Size Penalty 0,05, Window Size=500 or the length of the subject nucleotide sequence, whichever is shorter.

In a further preferred embodiment of the Invention the Internal nucleic acid quantity standard is generated by adding a defined quantity of a microbe or a mixture of microbes to the sample prior to lysis. The term "prior to lysis" refers to the state of the sample before the process of lysing the sample has been induced. However, if appropriate, the internal quantity standard can also be added at later stages, for example during lysis, after lysis, before nucleic acid extraction, after nucleic acid extraction, or during nucleic acid amplification. The term "defined quantity" refers to a countable or measurable number of microbes *in* the sample. If the microbe is a cellular microorganism, such as yeast, parasite, virus or bacteria, a cell culture may be grown and the cellular concentration can be obtained, for example, by using a coulter counter, a hemocytometer or a Neubauer counter chamber. Obtaining the concentration of virus particles present in a reference sample may be more difficult. However, if the number of protein subunits of a single virion are known, the virus particle concentration can be calculated from the protein concentration of the sample, The person skilled in the art knows several other methods of calculating the number of cells or virus particles present in a sample; see for example Maniatis, et al. Molecular Cloning: A laboratory manual, Cord Spring Harbor Press, New York, 1982 and Principles of Virology: Molecular Biology, Pathogenesis, and Control by S. Jane Flint, Amer Society for Microbiology.

In a preferred embodiment of the invention an internal DNA size standard is added to the sample. The DNA size standard contains at least one, preferably at least three, more preferably at least five, even more preferably at least seven and most preferred up to twenty DNA molecules of known length (in base pairs or nucleotides) and can be single stranded or double stranded. Alternative!, the internal size standard can also be an RNA molecule which is either single stranded or double In a preferred embodiment of the invention an internal DNA size standard is added to the sample. The DNA size standard contains at least one, preferably at least three, more preferably at least five, even more preferably at least seven and most preferred up to twenty DNA molecules of known length (in base pairs or nucleotides) and can be single stranded or double stranded. Alternatively, the internal size standard can also be an RNA molecule which is either single stranded or double stranded. Preferably, however, the internal size standard is a DNA molecule which is double stranded.

In a more preferred embodiment of the invention the internal size standard is labeled. The person skilled in the art knows a large number of labels that can be incorporated into nucleotides, either before or after polymerisation of the nucleic acid molecule present in the DNA standard. These include, for example, the abovementioned labeling compounds of primer oligonucleotides.

In another more preferred embodiment of the invention the Label of the size standard differs from the label of the oligonucleotides used for amplification of the DNA of the microbe. Different labeling of the size standard and amplified nucleic acid molecule results in a more straightforward identification of the amplified microbial nucleic acid molecule. Moreover, size standards containing nucleic acid molecules with various labels may be used, thus resulting in a better identification of the individual molecules of the size standard.

In a further more preferred embodiment of the invention the size standard is a mixture of DNA fragments in the range of 50 to 1600 bases. The size standard can be single stranded or double stranded. A single stranded size standard is used when the fragment length is determined under denaturing conditions while a double stranded size standard is used for non-denaturing conditions. Therefore, e.g., the term "50 to 1600 bases" refers to a double strand of up to 800 base pairs in length as well as to a single strand of up to 1600 bases in length. Further preferred mixtures contain DNA fragments in the range of about 50 to about 100, of about 50 to about 150, of about 50 to about 200, of about 50 to about 300, of about 50 to about 400, of about 50 to about 500, of about 50 to about 600, of about 50 to about 700 or of about 50 to about 750 bases or of 50 about 1600 base pairs. However, additional smaller or larger DNA fragments may be added to the standard mixture, for example DNA fragments of about 10, 20,30, 40 bases or base pairs in length.

In a further preferred embodiment of the invention the amplified DNA and/or the nucleic acid of the microbe in the sample is isolated and/or concentrated. The term "isolating" refers to the separation of nucleic acid molecules from other components present in a sample. The term "concentrated" means increase in amount per volume. Isolation and concentration are central steps in the purification of nucleic acid molecules and can be combined with further procedures in order to obtain a pure and concentrated nucleic acid sample. The Person skilled in the art knows various procedures for nucleic acid purification, e.g., electrophoretic methods, nucleic acid precipitation or silica purification; see for example Maniatis, et al. Molecular Cloning: A laboratory manual, Cold Spring Harbor Press, New York, 1982.

In another preferred embodiment of the Invention the nucleic acid of the microbe is amplified by RT-PCR, PCR, LCR, CPR, SDA, LAMP, NASBA,RCA, 3SR, QBR, bDNA. The microbial nucleic acid to be amplified is either RNA or DNA. Thus, the choice of the amplification method depends on the nature of the nucleic acid molecule. Before DNA amplification products can be generated from RNA molecules, the RNA has to be reverse transcribed into a DNA molecule. Avian myoblastosis virus (AMV) reverse transcriptase was the first widely used RNA-dependent DNA polymerase (Verma, 1977, Blochern. Biophys. Acta 473:1). The enzyme has $5^1$-$3^1$ RNA-directed DNA polymerase activity, $5^1$-$3'$ DNA-directed DNA polymerase activity, and RNase H activity. RNase is a processive 5' and 3' ribonuclease specific for the RNA strand of RNA-DNA hybrids (Perbal, 1984, A Practica] Guide to Molecular Cloning, Wiley & Sons New York). However, any other enzyme with reverse transcriptase activity can be used to transcribe the RNA molecule into a DNA molecule. The resulting DNA transcript can then be amplified by using appropriate primers and buffer conditions and by using, for example, any of the aforementioned DNA amplification methods.

In another preferred embodiment the DNA to be amplified comprises at least one variable domain flanked by at least two conserved domains. The term "conserved domain" or "conserved sequences" refers to a region in the genome of the microbe with relatively few variations. Nucleotide sequences are conserved, when their homology is exceeding 70% preferably 80%, more preferably exceeding 90%, even more preferably exceeding 95 and most preferred exceeding 99%. The term "variable domain" refers to a region in the genome of the microbe containing sequence variability or polymorphisms. Variable nucleotide sequences are characterized, e.g., by point mutations, deletions, substitutions or additions resulting in different restriction enzyme recognition sequences.

In a more preferred embodiment the oligonucleotides used for DNA amplification hybridize with the conserved sequences.

In another preferred embodiment the amplified nucleic acid contains nucleotides homologous to a nucleotide sequence selected from the group consisting of 16S rDNA, 23S rDNA, TUF, HSPs and US.

In yet another preferred embodiment the specific fragmentation is performed with at least one restriction enzyme. However, fragmentation can also be performed by using two, three or four restriction enzymes. When using more than one restriction enzyme, the cleavage reactions may be performed consecutively or simultaneously. Preferred restriction enzymes include those which recognize a recognition sequence of four or five nucleotides, including restriction enzymes selected from the group consisting of Aluf, Bsh12361, Bsp1431, Dpnl, Dpnll, FnuDll, Haelli, Hhal, Hpall, Maell, Mbol, Mspl, Malt], Rsal, Sau3A1, Taql, Tasf and Dpnl. Also preferred are restriction enzymes selected from the group consisting of Aatil, Boni, Bme13901, Bsp14071, Cfr131, Hinfl, Mn11, [Vivat, Hgal, Hphl, Mbol, StaNI, Taal and EcoPl. However, the fragmentation reaction may also be performed with one or more restriction enzymes selected from the group consisting of Aatil, Acc1, Acc651, Aci1, AclI, Afel, Aflll, Afllll, Agel, Ahdl, Alu1, Alwl, AlwNI, Apal, ApaL1, Apol, Asci, Asel, AsiS1, Aval, Avall, Avrll, Bael, BamHF, Banl, Banil, Bbsl, Bbvl, BbvCl, BceAl, Bcgl, BciVl, Ball, Bfal, BfrB!, BfuAl, Bgll, Bgill, Blpl, Bmel 5801, BmgBl, Bmrl, Bpml, Bsal, BsaAl, BsaBl, BsaHl, BsaJl, BsaWl, BsaXl, BseRI, Bsgl, BsiEl, BsiHKAl, BsiWF, Bs11, Bsmf, BsmAl, BsmBl, BsmFl, BsoBl, Bsp12861, BspCNl, BspDl, BspEl, BspHl, BspMl, Bsrl, BsrBl, BsrD1, BsrFl, BsrG1, BssHll, BssKl, BssSl, BstAPl, BstBl, BstE11, BstF51, BstNl, BstUl, BstX1, BstYl, BstZ171, Bsu361, Btgl, Btrl, Btsl, Cac8l, Clal, Ddel, Dpnl, Dpnll, Oral, Drall!, Drdl, Eaef, Eagl, Earl, Ectl, EcoN1, Eco01091, EcoRI, EcoRV, Faul, Fnu4HI, Fon Fsel, Fspl, **Hutt,** HaeIll, Hgal, Hhal, HinP11, Hindl, Hindill, Hinfl, Hpal, Hpall, Hphl, Hpy99I, Hpy1881, Hpy188111, HpyCH4I11, HpyCH41V, HpyCH4V, Kasi, Kpnl, Mbol, Mboll, Mfel, Miul, Mlyl, Mn11, Mscl, Msel, Msll, Mspl, MspA11, Mwol, Nael, Narl, Neil, Ncol, Ndel, NgoMIV, Nhel, NIaIll, NlalV, Notl, Nrul, Nsil, Nspl, Pacl, PaeR71, Peil, PflFI, PflMI, Plel, Pmel, Pmll, PpuMI, PshAl, Psil, PspGI, PspOMI, Pstl, Pvul, Pvull, Rsal, Rsrll, Sao!, Seen, San, Sapl, Sau961, Sau3Al, Sbfl, Scal, ScrFl, SexAl, SfaNI, Sfcl, Sfil, Sfol, SgrAl, Smal, Smll, SnaBl, Spel, Sphl, Sspl, Stul, Styl, Swal, Tagl, Tfil, Tlil, Tsel, Tsp451, Tsp5091, TspR1, Tth1111, Xbal, Xcml, Xhol, Xmal and Xmnl.

In another preferred embodiment the DNA fragment length is determined by DNA electrophoresis, HPLC, chromatography or mass spectrometry. Any method known to the Person skilled in the art is suitable for fragment length determination, provided the method allows to determine the length of at least one fragment of the amplified nucleic acid molecule with a precision of a single base or base pair. When using DNA electrophoresis as a means of determining the fragment length, the mixture containing the fragmented nucleic acid molecules is preferably separated on an automated sequencer device, as for example the 3100 Genetic Analyzer° automated sequencer. Preferably this device is equipped with a linear polymer matrix, such as the performance optimized polymer type 6 (Applied Biosystems). However, any other matrix is suitable as long as it provides the aforementioned precision.

In a more preferred embodiment of the Invention DNA electrophoresis is performed on a capillary sequencer, a multi-capillary sequencer or a high resolution polyacrylamide gel sequencer, or a microfluid based electrophoresis system. Preferably, the sample is loaded electrokinetically.

In another preferred embodiment of the invention more than one pair of oligonucleotides is used for nucleic acid amplification. By using several primer pairs, e.g. in a PCR reaction, several DNA fragments can be generated and analyzed, allowing the simultaneous detection of various different or closely related microbes. Furthermore, amplifying different fragments of the genome of a single microbe can result in an increased fidelity of the analysis.

In yet another preferred embodiment of the invention at least one nucleic acid fragment is amplified from one or more microbes.

In another preferred embodiment of the invention the microbe is selected from the group consisting of bacteria, protozoa, fungi, viruses and/or parasites.

In a more preferred embodiment of the invention the detection is species specific. A specie specific detection allows the detection of different species within the same genus. This allows, for example, the discrimination between streptomyces spp. and lactobacillus spp..

In a further preferred embodiment of the invention the sample represents a sample selected from the group consisting of water, dust, plant, soil, sputum, urine; blood, human or animal tissue. However, the term "sample" also refers to a sample taken from tissues, e.g., hair or skin, or body fluids, e.g., blood, saliva, semen, synovial fluid, amniotic fluid, breast milk, lymph, pulmonary sputum or surfactant or fecal matter.

In a further preferred embodiment of the Invention the method makes use of a databank storing the nucleic acid sequence information of the microbes and a computer program enabled to execute the following steps of: (a) searching in the data bank for sequences homologous to the oligonucleotides used for the nucleic acid amplification and copying homologous nucleotide sequences into a file; (b) searching for the presence of restriction enzyme recognition sequences within the nucleic acid sequences stored in the fite generated in step (a); and (c) performing an in silico restriction digest of said DNA by using the restriction enzyme recognition sequences of step (a)

In an embodiment, the disclosure provides a method for categorizing a vaginal microbiota community as normal, disturbed or vaginosis-related. Such a method involves identifying one or more predominant species of microbiota in a vaginal sample, thereby producing a vaginal microbiota profile; and, categorizing the vaginal microbiota population as normal, disturbed, vaginosis-related. Most commonly, the predominant species of microbiota is a species of bacteria, or a combination of species of bacteria. Nonetheless, the predominant species of microbiota can also include species of yeast, species of fungi, species of parasites and species of viruses.

In an embodiment, related profiles (that is, one or more consensus profiles) are identified using a clustering algorithm. A clustering algorithm is typically a statistical method, such as a cubical clustering criterion analysis, a pseudo F analysis or a pseudo T.sup.2 test, or a combination thereof.

In certain embodiments, the predominant species of vaginal microbiota is a species of bacteria selected from among *Lactobacillus crispatus, Lactobacillus iners, Lactobacillus jensenii, Lactobacillus gasseri, Lactobacillus coleohominis, Staphylococcus* sp., *Streptococcus* sp.; *Atopobium vaginae, Lachnospiraceae* sp., *Megasphaera* sp., *Enterococcus faecalis, Peptoniphilus* sp., *Anaerococcus* sp., *Micromonas* sp., *Gemella palaticanis, Dialister* sp., *Clostridaceae* sp. e.g., *Clostridium perfringens, Aerococcus* sp., *Veillonella* sp., *Finegoldia magna, Granulicatella elegans, Gardinerella vaginalis, Pseudomonas* sp., *Mycoplasma* sp., *Mobiluncus muleiri, Peptostreptococcus anaerobis,* Peptostreptococcus micros, *Eggerthella sp, Prevotella bivialP. buccalis,* Uncultured Prevotella, *'Leptotrichia amnionii', Dialister sp, Escherichia coli, Shigella* sp., or a bacterium of the order Clostridiales.

In another embodiment, a method for identifying at least one predominant species of microbiota indicating a normal, or disturbed or vaginosis-related microbiota in a vaginal sample is disclosed. Such a method for identifying predominant species of microbiota in a vaginal sample involves providing a vaginal sample comprising one or more species of vaginal microbiota, and detecting at least one predominant species of microbiota in the vaginal sample by a culture-independent method.

As described above, the predominant species of vaginal microbiota are typically identified using a culture-independent method of identification. For example, one or more predominant species of microbiota can be identified using PCR with selective primers, quantitative PCR with selective primers, DNA-DNA hybridization, RNA-DNA hybridization, in situ hybridization, any of a variety of comparable techniques, and combinations thereof. Optionally, DNA-DNA hybridization and/or RNA-DNA hybridization is performed on a microarray. In another embodiment, one or more predominant species of microbiota can be identified by determining the nucleotide sequence of a portion of a microbial genome, such as a 16S rRNA gene or other suitable genes.

The present invention also relates to a kit for the qualitative and/or quantitative detection of a microbe or for the simultaneous qualitative and quantitative detection of several different microbe comprising: at least one primer pair, consisting of a labeled and an unlabelled ollgonucleotide or at least two differently labeled oligonucleotides, to allow the amplification of the nucleic acid of the microbe; at least one nucleic acid or DNA quantity standard; in one or more containers and/or the above-mentioned computer program.

Finally, the present invention also relates to a diagnostic kit for the detection and categorization of vaginal microbiota. Based on the identification of species of microorganisms present in the vagina under normal health and pathological conditions, primers and probes are provided that greatly simplify the detection and categorization of vaginal microbiota. Using probes and/or primers specific for the species of microorganisms present in the vagina under normal, disturbed or vaginosis-related condition, the vaginal microbiota can be quickly and effectively categorized. Accordingly, primers and probes specific for the predominant species of normal and disturbed vaginal microbiota including the status of severe vaginosis are a feature of the invention. Similarly, kits including multiple primers and/or probes useful for detecting multiple species of microorganisms, such as the predominant species that define categories of normal, dysbalanced and vaginosis-related vaginal microbiota, are a feature of the invention. The primers and probes can be utilized in a wide variety of methods and protocols, including the hybridization and amplification methods described above and including T-RFLP as well. Optionally, the primers and probes can be immobilized onto a substrate, such as an array, e.g., a microarray on a glass or plastic matrix, chip or slide. Generally, the kits described herein are optionally packaged to include reagents for preparing nucleic acids or proteins, amplifying nucleic acids, and/or detecting nucleic acids or other biomolecules. For example, the kits optionally include assay components, such as buffers, reagents, enzymes, serum proteins (such as antibodies), receptors, etc., for detecting microorganisms normally present in the vagina. Optionally, additional probes and/or reagents are included for detecting pathological organisms such as yeast (Candida albicans), pathogenic bacteria, pathogenic parasites or viruses. In the case of prepackaged reagents, the kits optionally include pre-measured or pre-dosed reagents that are ready to incorporate into the assay methods without measurement, e.g., pre-measured fluid aliquots, or pre-weighed or pre-measured solid reagents that can be easily reconstituted by the end-user of the kit. Generally, such reagents are provided in a stabilized form, so as to prevent degradation or other loss during prolonged storage, e.g., from leakage. A number of stabilizing processes are widely used for reagents that are to be stored, such as the inclusion of chemical stabilizers (i.e., enzymatic inhibitors, microcides/bacteriostats, anticoagulants), the physical stabilization of the material, e.g., through immobilization on a solid support, entrapment in a matrix (i.e., a bead, a gel, etc.), lyophilization, or the like.

Such kits also typically include appropriate instructions for using the probes and reagents, and optionally for preparing samples and the like. The various elements of the kits are typically packaged together in a single package or set of related packages.

When utilizing a plurality of probes, for example, a plurality of probes each of which is specific for a species of normal, disturbed or vaginosis-related vaginal microbiota, such as those described in the Examples, the probes can be arranged in an ordered liquid or solid array. Optionally, the array also includes probes that are specific for pathogenic microorganisms (usually organisms that cause a pathology of the vagina). A wide variety of array formats can be employed in accordance with the present disclosure. One example includes a linear array of oligonucleotide bands, generally referred to in the art as a "dipstick." Another suitable format comprises a two-dimensional pattern of discrete cells (e.g., 96 squares in an 8 by 12 array). As would be readily appreciated by those skilled in the art, other array formats including, but not limited to, slot (rectangular) and circular arrays are equally suitable for use.

## EXAMPLES

### Example 1: Bacterial strains, quantification and model communities.

As a model microbial community *Pseudomonas aeruginosa* (ATCC 27853), *Staphylococcus aureus* (ATCC 29213), *Klebsiella pneumoniae* (ATCC 23357), *Burkhoideria cepacia* (clinical isolate), *Stenotrophomonas maltophilia* (clinical isolate), *Alcaligenes xylosoxidans* (clinical isolate) and *Mycobacterium avium* (clinical isolate) were used to determine reproducibility and optimal concentration of the amplicon in the PCR. Three bacteria were used to analyze the minimal microbial concentration that can be detected with our T-RFLP analysis process and evaluation of the quantitative standard were C. *jejuni* (same strain as used for the internal quantification and sizing standard), *P.aeruginosa* (ATCC 27853) and *Helicobacter pylori* (clinical isolate). Ali bacteria had been re-determined biochemically by the ATB system

(Bio Merieux) prior usage according to the manufacturer's instructions Bacterial strains were stored in the Microbank storage system (Pro-Lab Diagnostics) at -80°C. Prior to DNA extraction all seven bacteria of the model microbial community except *M. avium* had been cultured on Columbia agar (Becton Dickinson) overnight at 37°C. Several colonies from Columbia agar were transferred in 10 ml of brain heart infusion medium (Merck) separately and incubated at 37°C overnight. M. *avium* was cultured in Kirchner medium (BAG) overnight at 37°C from the frozen strain, directly. Thereafter, one ml of each bacterial suspension was transferred into the same 15 ml tube and mixed so that the tube contained 7 ml in total. 7 ml of PBS had been added to the tube and the resulting 14 ml of diluted suspension mixture were aliquoted in seven independent microbial community samples of 2 ml DNA

For the internal quantification standard *Campylobacter jejuni* (clinical isolate) was plated from frozen strains on Columbia agar (Becton Dickinson) directly and incubated overnight at 37°C under microaerophilic conditions. Colonies from C. *jejuni* were diluted in PBS (Gibco) containing 10% glycerol until an $OD_{600}$ rum = 0.4 ($1.5\ 10^6$ cfu/ml) [1], Several hundred aliquots of 100 pl from that dilution were made in 2 ml sample tubes and stored at -80°C.

### Example 2: Internal sizing standard and PCR conditions for the analysis of a 780 bp fragment and composites thereof out of the genomic *Campylobacter* DNA

For obtaining the additional fragment of the internal sizing standard *C. jejuni* (same strain as used for the internal quantification standard) was plated from fronen strains on Columbia agar (Betton Dickinson) directly and incubated overnight at 37°C under microaerophilic conditions. Colonies from C. *jejuni were* washed in 1 ml PBS. In addition to the DNA extraction protocol described for the other probes below genomic DNA of C. *jejuni* for this application was isolated by using a previousfy described protocol [2]. The 50 $\mu$l reaction mixture contained 5 $\mu$l of 10 x PCR buffer with 15 mM $MgCl_2$, 1 $\mu$l of a 25 mM $MgCl_2$ solution,4 % Glycerol (50%), 2.5 $\mu$l of a deoxynucleoside triphosphat mixture (10 mM each), 1 $\mu$l of each primer (100 pmol/pl), 1 arg C. *jejuni* genomic template DNA and 0.25 pl of DNA polymerase (5 U/$\mu$l).

All reagents except Glycerol (Sigma), dNTP mix (Roche Molecular Biochemicais) and primers (MWG Biotech) were obtained from Qiagen, Germany. The primers used for the amplification of the 780 bp 16S rDNA fragment (including primers) were UNIBAC 4F (5 "-CG GCGTGGACTACCAGGG-3 ") and UNIBAC 1R (5'AAGAGTTTGATCATGGCTC-3'). DNA amplification was performed with a model 2400 thermal cycier (Applied Biosystems, Foster City, CA) by using the foflowing program: 5 min at 95°C, followed by 35 cycles consisting of denaturation (25 s at 95°C), annealing (25 s at 50°C), extension (45 s at 72°C) and a final extension step at 72°C for 5 min. Amplified DNA was verified by electrophoresis of aliquots of PCR mixtures (5 $\mu$l) in 1.5% agarose in 0.5 x TBE buffer, For the PCR-product cleanup unincorporated primers, remaining genomic DNA and DNA polymerase were removed by using the QIAquick[rm] PCR Purification Kit (QIAGEN) according to the manufacturer's protocol. The resulting DNA solution was quantified at 260 nm and stored at 4°C.

### Example 3: Fragment cloning and Generation of fluorescently labeled 780 bp fragments and composites thereof from the plasmids

Fragment cloning was carried out with the TOPO TA Cloning[e] Kit for Sequencing Version E2 (Envitrogen) according to the manufacturer's protocol. Plasmids were isolated using the Q1Aprep® Miniprep Kit (Qiagen) according to the manufacturer's protocol. The purified plasmids were cycle-sequenced using the ABI PRISM® BigDye™ Terminator Cycle Sequencing Ready Reaction Kit (Applied Biosystems, Foster City, CA) according to the manufacturer's protocol to determine the accurate fragment length. Based an these data the best suited clone was selected to be the standard clone providing the 780 bp C. *jejuni* fragment elongating the GENESCAN® 500 ROX internal sizing standard, This done was then used for generating the desired 780 bp fragment. The remaining plasmid sofution of the standard clone was quantified at 260 nm and stored at 4°C until usage for PCR.

The 100 $\mu$l reaction mixture contained 10 $\mu l$ of 10 x PCR baffer with 20 mM $MgCl_2$, 8 $\mu$l of a deoxynucleoside triphosphate mixture (2.5 mM each), 1$\mu$l of each primer (10 pM), 35 ng genomic template DNA and 0.5 $pl$ of proofreading *TaKaRa Ex Tag[rm]* DNA polymerase (5 Utpl). All reagents except primers (MWG Biotech) were received from TAKARA SHUZO CO., LTD, Japan. The primers used for the amplification of the 780 bp 16S rDNA fragment from C. *jejuni* were UNIBAC 1R and UNIBAC 4F again, UNIBAC 1R was labeled with ROX at the 5'end. DNA amplification was performed with a model 2400 thermal cycler (Applied Biosystems, Foster City, CA) by using the following program: 3 min at 95°C, followed by 35 cycles consisting of denaturing (30 s at 95°C), annealing (30 s at 45°C), extension (45 s at 72°C) and a final extension step at 72°C for 7 min.

After the amplification unincorporated primers, remaining genomic DNA and DNA polymerase were removed by using the Q1Aquick™ PCR Purification Kit (QIAGEN) according to the manufacturer's protocol. The resulting DNA solution was quantified at 260 nm, diluted with HPLC grade water until a concentration of 12 ng/$\mu$l and stored at 4°C until usage,

### Example 4: Determination of the detectable concentrations

*P.aeruginosa* was plated on Columbia agar (Betton Dickinson) and incubated overnight at 37°C. Several colonies from *P.aeruginosa* were diluted in PBS to an

OD650 nm = 0.1 ($1.0 \cdot 10^5$ cfu/ml). From this suspension the following dilution steps were derived: $1.010^8$ cfu/ml, $1.010^7$ cfu/ml, $1.010^6$ cfu/ml, $1.0 \cdot 10^5$ cfu/ml, $1.0 \cdot 10^4$ cfu/ml. Additionally, suspensions with $1.010^9$ and $1.010^{10}$ cfu/ml were created and quantified by serial dilution. To characterize the precision of the method eight independent dilutions ranging from $1.010^{10}$ to $1.010^5$ were analyzed.

A second dilution was prepared with *H. pylori. H. pylori* was plated from frozen strains on Pylori Agar® (Bio Merieux) and incubated for 48 h at 37°C under microabrophilic conditions. Colonies of *H. pylori* were diluted in PBS to an OD600 nm 0.4 ($1.5 \cdot 10^9$ cfu/ml). From this suspension the following dilution steps were derived: $1.0 \cdot 1$ cfu/ml, $1.010^7$ cfu/ml, $1.0 \cdot 10^6$ cfu/ml, $1.010^5$ cfu/ml, $1.010^4$ cfu/ml, $1.0 \cdot 10^3$ cfu/ml, $1,0'10^2$ cfu/ml. To characterize the precision of the method eight independent dilutions ranging from $1.010^8$ to $1.010^4$ were analyzed.

One ml from each dilution step was transferred into a 2 ml sample tube containing the 100 $\mu$l of C. *jejuni* internal quantification standard. After that one ml of PBS was added to each tube resulting in a 2.1 ml suspension. The DNA of these seven dilution samples was extracted as described in the DNA extraction part. A real-time PCR (Ligth Cycler®; Roche Molecular Biochemicals) was then performed according to the manufacturer's instructions with the primers 8-27f and 1507-1492r as well as universal (6-FAM-TACGGCTACCTTGTTACGACTTCACCC-TAMRA-P) and *P.aeruginosa* specific (6-FAM-TAACCGTCCCCCTTGC-TAMRA-P) fluorescently labeled probes to determine both the absolute number of 16S copies and the 16S copies of each bacterial genus to compare the data with the results from the quantitative T-RFLP analysis by the internal quantitative standard.

Genomic DNA was isolated by using the following extraction protocol: The 2 ml of bacterial suspension derived as described above was centrifuged at 10.000g for 1 min in a pre-cooled centrifuge (4°C). The supernatant was discarded and 100 µl of lysis buffer (20 mM Tris-HCL (Roth), pH 8.0; 2 mM EDTA (Sigma); 1.2% Triton X100 as well as 40 ml lysozyme solution (100 mg/ml; Sigma) and 40 µl lysostaphin solution (1 mg/ml; Sigma) were added to the sample, mixed and incubated for 1.5 h at 37°C. After that 20 µl of a proteinase K solution (20 mg/ml; Qiagen) was added and incubated for 1 h at 56°C. Inactivation of the enzymes was performed by heating the samples up to 95°C for 5 min. To remove RNA 20 µl RNase (20 mg/ml; Boehringer Mannheim) was added to the samples, mixed and incubated at 37°C for 30 min. Then 200 µl of buffer AL (Qiagen) was added to the samples and DNA was prepared as described by the manufacturer (Qiagen, Hilden, Germany) using the blood mini kit. The resulting DNA solution was quantified at 260 nm and stored at 4°C. Several bacterial genera (e.g. C. *jejuni, P.aeruginosa, S. aureus* etc.) were plated on their corresponding media and cultivated under the growing conditions described above. Genomic DNA was isolated using the QJAamp™ DNA Mini Kit (Qiagen). Four dilutions of the isolated DNA (1:10, 1:100, 1: 1000, 1: 10000) were obtained and co-amplified in a real-time PCR (Ligth Cycler®; Roche Molecular Biochemicals) with a plasmid harbouring the single 16S gene inserted. For detection, a fluorescently labeled and universal probe (6-FAM-GCAAACAGGATTAGATACCCTGGTAGTC-TAMRA-P) was added to the reaction mixture. The siope in the linear range of the amplification curve was calculated by the Light Cycler® Data Analysis Software; its antilogarithm represents the amplification efficiency for the specified bacterial genome size and the adjusted PCR conditions.

The 50 µl reaction mixture contained 5 µl *of* 10 x PCR buffer with 20 mM MgCl$_2$, 4 µl of a deoxynucleoside triphosphate mixture (2.5 mM each), 0.5 µl *of* each primer (10 *pM),* 0.5 µl of a BSA solution (10 mg/ml), 30 ng of genomic template DNA for determining the efficiency of the T-RFLP technique/reproduction of the results and further of the minimal detectable concentration/ operativeness of the internal quantitation standard (55, 50, 30, 25, 10, 5 and 1 ng for determining the optimal amplicon concentration) and 0.25 µl of proofreading *TaKaRa Ex Tag$^{dm}$* DNA polymerase (5 U/µl). All reagents except BSA solution (New England Biolabs) and primers (MWG Biotech) were received from TAKARA SHUZO CO., LTD, Japan. The primers used for the amplification of nearly the complete 16S rDNA gene were 8-27f labeled with FAM (5-carboxyfluorescein) (5"-AGAGTTTGATCCTGGCTCAG3') [4] and 1507-1492r unlabeled (5"-TACCTTGTTACGACTT-3') [5]. DNA amplification was performed with a model 2400 thermal cycler (Applied Biosystems, Foster City, CA): 2 min at 94°C, followed by 35 cycles consisting of annealing (30 s at 50°C), extension (1 min at 72°C), denaturation (10 s at 94°C) and a final annealing step at 55°C for 1 min as well as a final extension step at 72°C for 5 min. For the cleanup of the amplicon the total amount (50 µl) of amplified DNA was mixed with 10 µl of 6x concentrated electrophoresis buffer (Helena Biosciences) and separated by electrophoresis an 1.5% agarose (Gibco) in 0.5 x TBE buffer at 150 Volt. DNA bands were excised and purified using the Q1Aquick™ Gel Extraction Kit (Qiagen) according to the manufacturer's protocol. The elution volume was 30 µl of EB buffer (Qiagen). Finally, the purified PCR products were also cycle-sequenced to determine their accurate fragment length arid identity using the ABI PRISM° BigDye™ Terminator Cycle Sequencing Ready Reaction Kit (Applied Biosystems, Foster City, CA) according to the manufacturer's protocol.

To obtain the optimal amount of template for a successful species determination PCR reactions with 55-, 50-, 30-, 25-, 5- and 1 ng of DNA were produced. Each of these concentration steps was performed seven times with DNA from independent extractions. Fluorescence signals increased up to 30 ng of template in a 50 pl reaction. Higher DNA concentrations resulted in reduced signals, probably due to template inhibition effects. Thereby, the ratio of a single bacterial fraction and the bacterial species itself in this total template amount was not of importance. Species with low concentrations (e.g, *K. pneumoniae)* showed the same signal distribution than those with high cell numbers when the microbial community model was applied (e.g. *M. avium).* Thus, a direct comparison among different peaks within a single run is possible.

**Example 6: Amplicon digestion and analysis of T-RFLP profiles**

A 20 µl of digestion mixture was prepared containing 10 pl of purified amplicon (volume is important not concentration), 2 µl of 10x NEBuffer 4, 1 µl of *Hha* solution (20,000 U/ml), 0.2 µl of a 100x BSA solution (10 mg/ml) and 6.8 µl water (HPLC grade). All reagents except the HPLC grade water (Roth) were received from New England Biolabs. Digestion was performed in 0.2 ml thin walled tubes with a model 2400 thermal cycler (Applied Biosystems, Foster City, CA) for 3 h at 37°C. The precise lengths of the T-RFLP fragments from the amplified rDNA products were determined by multi-capillary electrophoresis with a model 3100 Genetic Analyzer automated sequencer (Applied Biosystems, Foster City, CA), as follows. 3 µl of the digested DNA were mixed with 20 µl Hi-Di formamide solution (Applied Biosystems), 1 µl *GENESCAN*® 500 ROX sizing standard (Applied Biosystems) and 1 µl 780 bp *C. jejuni* fragment (12 ng/µl) produced as described above. The sizes of the GENESCAN® 500 ROX sizing standard were 35-, 50-, 75-, 100-, 139-, 150-, 160-, 200-, 300-, 350-, 400-, 450-, 490-, and 500 bp respectively. The mixture was then separated on the 3100 Genetic Analyzer[e] automated sequencer in sequencing mode of the data collection software® with the following run parameters: run temperature 50°C, capillary fill voltage 184 steps, pre run voltage 244 V/cm, pre run time 180 sec, injection voltage 30 V/cm, injection time 20 sec, run voltage 244 V/cm, data delay time 1200 sec, and run time 6,500 sec. The linear polymer matrix consisted of the performance optimized polymer type 6 (Applied Biosystems). After electrophoresis the produced sequencing Eifes (.abl format) were readjusted in GENESCAN® flies (.fsa format). The lengths of the FAM fluorescently labeled fragments were determined by comparison with the new ROX fluorescently labeled internal size standard consisting of the GENESCAN® 500 ROX and the generated 780 bp C. *jejuni* fragment by using the GENESCAN° Analysis software 37 with the following analysis parameters: analysis range 400 to 11,000 data points, light smooth option, peak amplitude threshold for all dyes 50 fluorescence units, minimal peak half width 2 pts., polynomial degree 3, peak window size 19 pts., slope threshold for peak start 0.0, slope threshold for peak end 0.0, full size call range, local Southern algorithm as size calling method, baselining 251 pts. The determination of the bacteria corresponding to the detected peak sizes was performed using the T-RFLP analysis program (TAP) and Ribosomal Database of the Center for Microbial Ecology at Michigan State University (http://rdp.eme.msu.edu/htmliTAP-trflp.html#program). The 8-27f primer was Input as the forward primer and 5"-GCGC-3' as the *Hha* 1 restriction enzyme target sequence. The maximum number of Oase mismatches allowed within the specified number of bases from the 5'end of the 8-27f primer was zero.

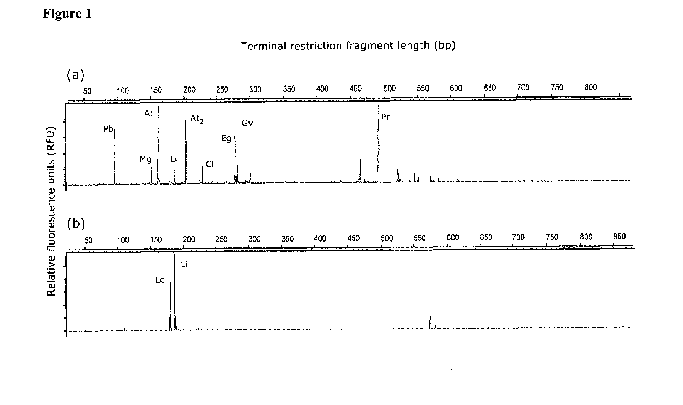

In a first set of experiments a model microbial community was prepared as described in Materials and Methods consisting of seven distinct clinical relevant microbial species. Figure 1 demonstrates the T-RFLP peaks from seven independent DNA isolations of the same model microbial community after *Hha* 1 digestion. These seven peaks of each of the diverse bacterial species were laid an top of each other to Show the accurate fragment sizing with our self-defined 780 bp or longer single stranded ROX labeled sizing standard. Table 1 summarizes the data for the seven bacterial strains. All peaks are very close to the size that is calculated by the TAP T-RFLP analysis program for the above species [6]. The nominal fragment size after *Hha 1* digestion of the single bacterial species used was examined by sequence analysis, too. The difference from the nominal size was less than half a base pair. Therefore, an accurate fragment sizing with ±1 bp necessary for an unknown microorganism was always possible by choosing POP 6 (Applied Biosystems) as the sieving matrix and separating in sequencing mode with reduced run voltage (244 V/cm instead of 300 V/cm). Further, the peaks well contrasted with the natural fluorescence background and were able to be reproduced. Therefore, both the fundamental operativeness of our T-RFLP technique and the ability of reproduction of the results as well as the feasibility of the self-created internal sizing standard could be determined. The DNA isolation technique especially developed for the T-RFLP application is suitable to completely lyse the rigid gram positive and mycobacterial cell walls without coequally destroying the relatively fragile gram negative bacteria and their genomic DNA. *P.aeruginosa, B. cepacia* and A.

*xylosoxidans* form twin-peaks resulting most likely from isoforms of the 16S rDNA. Thus, our data show that, if bacteria generate twin-peaks, the right hand side peak corresponds to the accurate fragment sizing.

**Example 7: Quantification of the T-RFLP**

For the quantification of the T-RFLP results an internal quantification standard with approximately $6.4 \times 10^5$ cells of C. *jejuni* (approximately two million 16S rDNA copies) was used for each DNA extraction probe. This internal quantification standard is necessary since the penetration for laser and fluorophore emission light among the different capillaries of a multi-capillary array is manufacture dependent. An external characteristic curve will lead to significant measurement errors. Due to the appearance of twin-peaks and the failure of a Gaussian shape of the peaks the peak area must be taken always when T-RFLP analysis an capillary sequencers with multi-fluorophore laser-scanning DNA detection (MLSDD) is performed. A simple linear comparison among different peak areas cannot be expected because the PCR analysis results in an exponential amplification of a DNA segment specified by two specific oligonucleotide primers. The relationship between the number of copies before and after amplification can be expressed in an equation as follows [7]:

$$N = s * (f)^n \qquad (1)$$

where n is the number of cycles of PCR completed, s the number of copies of a DNA segment present at the start of the reaction, N the number of copies present after n cycles of amplification, and f the efficiency of amplification.

Equation (1) is more conveniently written by converting to natural logarithms:

$$\ln(N) = n * [\ln(f)] + \ln(s) \qquad (2)$$

Equation (2) expresses a linear relation between n and In N. The line's slope is In (f), the natural logarithm of the efficiency, and its intercept is in (s), the natural logarithm of starting amount. In theory In N could be plotted versus n. Then in (s), and therefore s, could be estimated by linear regression and extrapolation. In case the number of cells from which s was obtained is known, dividing by the number of cells would yield the number of copies of s per cell. However, there is one problem with this approach in practice. The number of cells must be known accurately. To overcome this problem, a reference 16S rDNA gene (internal standard) can be simultaneously amplified (co-amplified) with the other 16S rDNA genes in the microbial community. Equation (2) applies to the genes of interest (Index i) as well as the reference gene (index r). So, the ratio of $s_i$, the starting number of copies of the gene of interest, to sr, the starting number of the reference gene should be independent of the number of cells from which and $s_r$ are obtained. In this situation the following equation will be obtained:

$$ln\left(\frac{s_i}{s_r}\right) = ln\left(\frac{N_i}{N_r}\right) - n * \left[ln\left(\frac{f_i}{f_r}\right)\right] \qquad (3)$$

With In ($s_i$/sr) = ln($s_i$) - ln($s_r$) rearranging of equation (3) yields:

$$ln(s_i) = ln\left(\frac{N_i}{N_r}\right) - n * \left[ln\left(\frac{f_i}{f_r}\right)\right] + ln(s_r) = \qquad (4)$$

Assuming ln($N_i$/$N_r$) In ($A_i$/$A_r$), with A as the peak area in the electropherograms, we get:

$$ln(s_i) = ln\left(\frac{A_i}{A_r}\right) - n * \left[ln\left(\frac{f_i}{f_r}\right)\right] + ln(s_r) = \qquad (5)$$

The antilogarithm of In $s_i$ is the number of copies of the 16S rDNA genes of interest. To obtain the total number of procaryotic cells s has to be divided by the genus specific number of the 16S rDNA genes per cell. The ratio between $f_i$ and fr is orte of the most important terms in the equation above (5). From our data it can be supposed that the efficiency of a given PCR system for different genomic templates depends on the size of the genomes. Large genomes impair the access to the annealing sites for the primers. The efficiency for a given PCR system and genomic template can be obtained by protracting the number of copies per cycle versus the number of cycles. The slope of the linear range is the natural logarithm of the efficiency. Thus, the efficiency f is the antilogarithm of the slope. Since the efficiencies for all bacteria which may appear within a model or natural community cannot be separately determined for our PCR system we determified and interpolated the efficiencies for several bacterial genera with genome sizes ranging from 0.5 Mbp to 6.5 Mbp using a real time PCR (table 2). It is evident that the efficiencies decrease with increasing genome size. When working with the listed efficiencies, variations may be possible, occasionally. In the majority of cases they should work enabling a sufficient quantification accuracy besides real time PCR technology.

Another important term of equation (5) is the number of total cycles in the PCR. As mentioned above the accumulation of copies follows a logarithmic function. But in practice after a certain number of cycles (mostly in the range of 33 to 35) the graph converges against a horizontal asymptote. Therefore, a linear relation between cycle number and copy number cannot be assumed (plateau effect). Conveniently, this can be avoided by decreasing the cycle numbers between 18 and 28. In addition, lower amounts of the starting template can help to keep this linear relation,

Under this consideration, we only used 30 ng of total amplicon amount per PCR reaction and performed 35 cycles wilhout loosing quantification ability. This was also confirmed by real time PCR (data not shown),

**Example 8: range and the detectable bacterial concentrations**

To characterize the range and the detectable bacterial concentrations of our TRFLP technique two different serial dilutions were applied from $1,010^{10}$ to $1.010^3$ cfu/rnl of *P. aeruginosa* and $1.010^8$ to $1,010^3$ cfu/ml of *H. pylori,* respectively. The internal quantification standard with the reference 16S rDNA genes was a bacterial suspension of 6.4 • $10^5$ cfu of *C. jejuni* corresponding to 2 million copies of 16S rDNA. Table 3 shows the results of the T-RFLP analysis for *P. aeruginosa* as an example for a bacterium with a large genome size resulting in a reduced amplification efficiency. It is evident that *P.aeruginosa* (6,264,403 bp) can be detected in a wide range from $1.010^{10}$ to $1.0$- $10^5$ 16S copies per sample (2.1 ml final volume) when using *C. jejuni* (1,641,481 bp) as the internal quantification standard. For the quantification with equation (5) one has to note that the relatively small genome size of *C. jejuni* (f = 1.150) does not affect the amplification of the 16S rDNA genes in a similar manner equally compared to *P.aeruginosa* (f =1.030). The data show that the peak ratio decreases with lower concentrations. Additionally, the precision of the method was characterized by eight independent serial dilutions of the lower ($1.010^5$ cfu) and the upper detection limit ($1.010^{10}$ cfu). The standard error was 2.5% and 2.1% of the sample mean, respectively. The upper detection limit was drawn by the linear detection range of the CCD camera. Because the total DNA amount always kept at 30 ng the relative peak area for C. *jejuni* continuously increases with the increasing *P. aeruginosa* dilution (figure 2). Table 4 shows that *H. pylori* (1,667,867 bp) can also be detected in a wide range from $10^8$ to $10^4$ 16S copy numbers per probe (2.1 ml final volume) when using *C. jejuni* (1,641,481 bp) as the internal quantification standard. The similar genome sizes of *H. pylori* (f = 1.150) and C. *jejuni* f =1.150) affect the amplification of the 16S rDNA genes in a comparable way. So, the ratio of the efficiencies in equation (5) is one. Eight independent serial dilutions gave a standard error of 2.7% ($1.010^4$ cfu) and 0.8%

($1.010^8$ cfu) of the sample mean, respectively. In all cases the precision (repeatability) of the quantification was better than 3%. To investigate the Performance of the T-RFLP method all samples were additionally characterized by real time PCR (figure 3 and 4). The real time PCR is a comparable quantification technique. However, in contrast to the T-RFLP, that allows to quantify a complex community, the real time PCR is limited to the quantification of only one organism at a time. So, DNA extracts from single bacterial cultures were produced and

applied in a real time PCR process. The data from both the T-RFLP and the real time PCR was statistically compared. Linear regression leads to a slope of 0.9995 (P. *aeruginosa*) and 0.9903 *(1-1. pylon),* The intercept is -0.02 *(P. aeruginosa)* and 0.0489 *(H. pylon).* In both cases the correlation coefficient is 0.9999.

## Example 9: Analysis of the vaginal bacterial community not associated with BV as well as associated with BV

Cultivation-independent methods were used to analyze samples collected at multiple time points from the mid-vagina of a total of 126 women without (n=76) and with clinical signs (n=50) of bacterial vaginosis. The species composition and structure (proportion of each species) of each community was assessed.

In brief, total microbial community DNA was isolated from microbial cells that had been retrieved on vaginal swabs. Genomic DNA was isolated from 0.5 ml aliquots of the cell suspensions as described above in detail. Samples were prepared for T-RFLP analysis as described above. The various T-RFs were distinguished on the basis of differences in fluorescence. 16S rRNA gene libraries were prepared from representative samples to identify the numerically dominant constituent populations. To construct the libraries, 3 μl of PCR product was cloned in a TOPO vector (TOPO TA cloning kit, Invitrogen, San Diego, Calif., USA) using the method recommended by the manufacturer except that salts, nucleotides and primers were first removed from PCR products using Qiaquick PCR purification kits (Qiagen). One Shot *E. coli* cells (Invitrogen) were transformed with ligated plasmids and 50 μl of each transformation mixture was spread onto Luria-Bertani (LB) agar plates that contained X-gal, IPTG, and 50 μg/ml kanamycin. After incubation overnight at 37° C., 100 white colonies were picked, inoculated into 5 ml aliquots of LB broth that contained 50 μg/ml kanamycin. After being incubated overnight at 37° C., the cells were then harvested from each culture and plasmids were extracted. The 16S rRNA gene inserts were individually amplified by PCR using the conditions described above, and the amplified 16S rRNA gene inserts were subjected to terminal restriction fragment analysis as described above. Clones yielding T-RFs that corresponded to those in the T-RFLP profile were sequenced and the data were analyzed as described above.

From each library, the DNA sequences of approximately 200 16S rRNA clones were determined and subjected to phylogenetic analyses. The sequences of the 16S rRNA genes of reference strains were determined with 4 cycle sequencing reactions. Each sequencing reaction contained 4 μl of 5x Sequencing Buffer, 2 μl of the Ready Reaction Mix (Applied Biosystems Instruments, Foster City, Calif., USA), 20 ng of template DNA, and a final concentration of 0.2 μM of primer. Sterile water was added to a final volume of 20 μl. Each cycle sequencing reaction was comprised of 25 cycles and each cycle included a melting step at 96° C. (10 sec), followed by primer annealing at 50° C. (5 sec), and extension at 60° C. (4 min). Prior to sequence analysis, the products were purified using the isopropanol precipitation method as described by ABI. Sequence data were collected using an ABI Prism 3100 Genetic Analyzer, and analyzed using the appropriate software (ABI). The 16S rRNA gene sequences obtained were matched with all sequences presently available from the databases of the Ribosomal Database Project (RDP), and GENBANK® to identify their closest relatives. The relatedness of communities was characterized using the software program Cluster, which groups profiles based on the number, size, and abundance of 5' and 3' fragments in each profile. The software program Treeview was used to visualize the relationships between samples as dendrograms. The similarity tree was generated using average linkage clustering with an uncentered correlation metric. These applications for Windows operating systems are available on the world wide web at rana.lbl.gov/EisenSoftware.htm.

Marked differences in the diversity and species of organisms detected in the vaginal microbial communities were observed in vaginal samples from healthy compared to vaginal samples from women with clinical sign of vaginosis.

In the 50 BV samples, T-RFLP displayed a high level of bacterial diversity. Twenty-three different T-RF patterns corresponding to 'operational taxonomic units' (further named bacterial species or phylotypes) were identified unambiguously. On average, in each BV sample, 6,3T-RF patterns were found (range 2-14). The frequency at which each of these 23 species or phylotypes occurred in the BV sample set ranged from 96% *(Atopobium vaginae,* the dominating bacterium in our BV samples) to 2% *(Lactobacillus crispatus,* present in a single sample). An uncultured *Megasphaera sp., Lactobacillus iners* and G. *vaginalis* were frequently detected (68, 64 and 64 %, respectively). The 16S rRNA gene sequence with the most significant RF score for the Megasphaera T-RF pattern was GenBank accession number AY959093, which showed highest similarity (95 %), among all unambiguously annotated sequences, to the Megasphaera elsdenii 16S rRNA gene. Several bacterial species or phylotypes occurred with moderate relative frequencies from 44 to 10 %: *Eggerthella sp.,* several Clostridium-like and *Prevotella spp., Peptostreptococcus (Micromonas) micros, Aerococcus christensenii, Leptotrichia amnionii, Peptoniphilus sp., Dialister sp., Mycoplasma hominis* and bacteria from the family *Enterobacteriaceae.* The three clostridial T-RFs showed highest concordance with the sequences of GenBank accession numbers AY958888, AY959097 and AY995273, respectively. The corresponding bacteria were designated bacterial vaginosis associated bacterium 2 (BVAB2), BVAB1 and BVAB3 by Fredricks et al. (2005), as they were only distantly related to known clostridia (16S rRNA gene identity ~90 %). In our BV sample set, BVAB2 occurred with highest frequency (36 %), followed by BVAB1 (18 %). BVAB3 was only rarely detected (4 %). In addition to the aforementioned bacteria, five further species were identified in 10% of all specimens: Sneathia sanguinegens, Anaerococcus tetradius, *Mobiluncus sp., Finegoldia magna* and *Lactobacillus crispatus.*

**Example 10: Analysis of the vaginal bacterial community in pregnant women.**

Cultivation-independent methods were used to analyze samples collected during pregnancy from week 34 to 41 from the mid-vagina of a total of 50 women without clinical signs (n=50) for preterm delivery. The species composition and structure (proportion of each species) of each community was assessed. The technical procedure was as described above. 62% of the pregnant women hat a lactobacillus dominated microbiota without the presence of additional bacteria such as *enterobacteriaceae, staphylococus aureus* or *enterococcus spp.* In consequence 38% of the pregnant women had a mixed microbiota without the presence of vaginose-associated microorganism. Pregnant women with a lactobacillus dominat flora harboured predominantly *lactobacillus crispatus, L. jensenii, L. gasseri* and *L. acidophilus.* The appearance of *L. iners* alone or in addition to other *lactobacillus spp.* is characterized by a disturbance of the vagina microbiota

Table 1: Sizing precision after T-RFLP analysis on the multi-capillary 3100 Genetic Analyzer. Difference between average number of base pairs and nominal number of base pairs for all seven determined prokaryotic genera is less than 0.5 base pair. For the analysis of bacteria that generate twin-peaks (*) the one on the right hand side was used for accurate sizing.

| Probe No. | *P. aeruginosa** | *B. cepacia** | *S maltophila* | S aureus | *M avium* | K pneumoniae | xylosoxyclans* |
|---|---|---|---|---|---|---|---|
| 1 | 154.89 | 202.22 | 212.84 | 237.60 | 367.09 | 37L64 | 567.47 |
| 2 | 154.83 | 202.19 | 212.88 | 237,73 | 366.91 | 371.45 | 567.41 |
| 3 | 154.89 | 202.28 | 212.92 | 237.71 | 367.02 | 371.52 | 567.51 |
| 4 | 154.92 | 20229 | 212.84 | 237.65 | 367.09 | 371.56 | 567.62 |
| 5 | 154.86 | 202.20 | 212.90 | 237.72 | 366.88 | 371.47 | 56733 |
| 6 | 154.86 | 202.21 | 212.83 | 237.78 | 366.99 | 371.55 | 567.77 |
| 7 | 154.80 | 20221 | 212.88 | 237.68 | 367.00 | 371.58 | 567.51 |
| average number of bp | 154.86 | 20223 | 212.87 | 237.70 | 366.98 | 371.54 | 567.52 |
| nominal number of bp | 155,00 | 202,00 | 213.00 | 238,00 | 367.00 | 371.00 | 567.07 |

Table 2: Correlation of bacterial genome size and PCR efficiency with primers FAM-8f and 1492r under conditions described in the materials and methods pari. The efficiency range was estimated from data obtained of several bacterial genera by real time PCR technology-

| genome size [Mbp] | | efficiency range [-] | |
|---|---|---|---|
| 0.5 | -0.5 | 1.15 | - 1.13 |
| 1.5 | -2.5 | 1.13 | - 1.11 |
| 2.5 | -3.5 | 1.11 | - 1.09 |
| 3.5 | -4.5 | 1.09 | - 1.07 |

(continued)

| genome size [Mbp] | | efficiency range [-] | |
|---|---|---|---|
| 4.5 | -5.5 | 1.07 | - 1.05 |
| ... | | | |
| 5.5 | -6.5 | 1.05 | - 1.03 |

Table 3: Range and detection limit of the T-RFLP analysis for a bacterium (*P. aeruginosa)* with a reduced amplification efficiency compared to the interne! quantification standard. In the average one cfu of *C. jejuni* corresponds to three 16S copies and on cfu of *P. aeruginosa* to four 16S copies, respectively. The number of 16S copies for C. *jefuni* was aiways adjusted to $2.0 10^6$. Sample volume: 2.1 ml bacteria/ appr.

| number of cfu/probe | peak area in T-RFLP analysis | peak ratio H | number of starting 16S copies per probe calculated with | number of starting 16S copies per probe measured by real time PCR s[-J | deviation As TᵉA] |
|---|---|---|---|---|---|
| *C. jejunil* $6.4 10^5$ | 101 | 394.17 | | - | |
| P. aeruginosa/ $1.0 \cdot 10^{10}$ | 39811 | | $3.73\ 10^{10}$ | $3.82\ 10^{10}$ | -2.36 |
| *C. jejunil* $6.4\ 10^5$ | 699 | 35.97 | - | | - |
| *P. aeruginosal* $1.0\ 10^8$ | 25145 | | $3.41\text{-}10^9$ | $3.66 \cdot 10^9$ | - 6.83 |
| *C. jejunk* $6.4 10^5$ | 3812 | 3.98 | | | |
| *P. aeruginosal* $1.0\text{-}10^8$ | 15172 | | $3.77\text{-}10^8$ | $4.02 10^8$ | -6.22 |
| *C. jejunil* $6.4\ 10^5$ | 8002 | 0.37 | - | - | - |
| *P. aeruginosal* $1.0 10^7$ | 2978 | | $3.52\text{-}10^7$ | $384 \cdot 10^7$ | -8.34 |
| *C. fejunil* $6.4\text{-}10^5$ | 17337 | 0.043 | - | | |
| *P. aeruginosal* $1.0 \cdot 10^6$ | 743 | | $4.06\text{-}10^8$ | $4.37 \cdot 10^6$ | -7.09 |
| *C. jejunil* $6,4 10^5$ | 3574] | 0.004197 | - | | - |
| *P. aeruginosal* $1,0\text{-}10^5$ | 150 | | $3.97 \cdot 10^5$ | $4.01\ 10^5$ | -1.00 |

Table 4: Range and detection limit of the T-RFLP analysis for a bacterium *(H. pylori)* with nearly the same amplification efficiency compared to the internal quantification standard (C. *jejuni).* In the average one cfu of C. *jejuni* corresponds to three 16S copies and one du of *H. pylori* to two 16S copies, respectively. The number of 16S copies for *C jejuni* was always adjusted to $2.0'10^6$. Sample volurne 2,1 ml.

| bacteriai appr. number of cfu/probe | peak area in A[..] | peak ratio [-] | number of startMg 16S copies per probe calculated s[-] | number of starting 16S copies per probe measured | deviation As [Z] |
|---|---|---|---|---|---|
| *C. jejuni/* $6.4 \cdot 10 5$ | 423 | 93.34 | - | - | - |
| *H. pyloril* $1 \cdot 10^9$ | 39481 | | $1.87\ 10^8$ | $1.99 \cdot 10'$ | -6.03 |

(continued)

| bacteriai appr. number of cfu/probe | peak area in A[..] | peak ratio [-] | number of startMg 16S copies per probe calculated s[-] | number of starting 16S copies per probe measured | deviation As [Z] |
|---|---|---|---|---|---|
| *C. jejunil* 6.4·10⁵ *H. pyloril* 110[1] | 2243 20196 | 9.42 | $1.8810^1$ | $1.9740^7$ | - -4.57 |
| *C. jejunil* 6.4·1.0⁵ *H. pyloril* 1·10⁶ | 5981 5299 | 0.88 | - $1.7710^6$ | - $1{,}81\cdot10^6$ | -7.11 |
| *C. jejunil* 6.410⁵ *H. pyloril* 1·10⁵ | 25133 2210 | 0.088 | - $1.7610^5$ | - $1.88\cdot10^5$ | - -6.38 |
| *C jejunif* 6.4-0⁵ *-1. pyloril* 1·10' | 39573 409 | 0.010335 | ... $2.0710^4$ | - $1.95\text{-}10^4$ | - -1-6.15 |

Table 5: Abundance of phylotypes in 16S rRNA gene clone libraries of non-BV and BV associated communities.

| Phylotype/species | BV samples (n=50) | Non-BV Samples (n=76) |
|---|---|---|
| *Atopobium vaginae* | 48 | |
| *Megasphaera sp.* (GenBank no. AY959093) | 34 | |
| *Lactobacillus iners* | 32 | 26 |
| *Gardnerella vaginalis* | 32 | |
| *Eggerthella sp* | 22 | |
| *Clostridium-like sp.* (GenBank no. AY958888) | 18 | |
| *Prevotella bivia* group | 18 | |
| *Peptostreptococcus micros* | 17 | |
| *Prevotella sp.* (GenBank no. AY959212) | 15 | |
| *Aerococcus christensenii* | 12 | |
| *Leptotrichia amnionii'* | 9 | |
| Peptoniphilus sp. | 9 | |
| Bacterium from the order Clostridiales (GenBank no. AY959097) | 8 | |
| *Dialister sp* | 8 | |
| *Prevotella buccalis* group | 8 | |
| *Enterobacteriaceae* | 5 | |
| *Mycoplasma hominis* | 5 | |
| *Sneathia sanguinegens* | 3 | |
| *Anaerococcus tetradius* | 3 | |
| *Clostridium-like sp.* (GenBank no. AY995273) | 2 | |
| *Mobiluncus sp* | 2 | |
| *Finegoldia magna* | 2 | |
| *Lactobacillus crispatus* group | 1 | 33 |

(continued)

| Phylotype/species | BV samples (n=50) | Non-BV Samples (n=76) |
|---|---|---|
| *Lactobacillus gasseri* group | | 8 |
| *Lactobacillus jenseni* | | 7 |
| *Lactobacillus johnsonii* | | 1 |
| *Lactobacillus acidophilus* | | 1 |

Phylogenetically related clones that on average had 90% sequence similarity to a reference strain were presumed to be of the same genus, and clones that on average had 97% sequence similarity were designated with the corresponding epithet. Clones with <90% similarity to known organisms were considered to be novel.

Table 5: Abundance of *Lactobacillus species* in 16S rRNA gene clone libraries of pregnancy associated communities (n=50).

| Phylotype/species | healthy microbiota | Disturbed microbiota* |
|---|---|---|
| *Lactobacillus iners* | 9 | 11 |
| *Lactobacillus crispatus group* | 19 | 6 |
| *Lactobacillus gasseri group* | 5 | 2 |
| *Lactobacillus jenseni* | 5 | 4 |
| *Lactobacillus johnsonii* | 0 | 1 |
| *Lactobacillus acidophilus* | 1 | 0 |
| *Characterized by the presence of significant amounts of *staphylokokkus aureus, enterococcus sp., Escherichia coli.* | | |

**Figure 1** Examples of T-RFLP patterns from vaginal smears. The 16S rRNA gene was amplified with primers 27F and 926R and amplicons were digested with *Msp*I. Only 6-FAM-labelled T-RFs are displayed. (a) Bacterial vaginosis sample; (b) normal sample. Pb, *P. bivia*; Mg, *Megasphaera* sp.; At, *Atopobium vaginae*; Li, *Lactobacillus iners;* At$_2$, *Atopobium vaginae* (incomplete digestion); Cl, *Clostridium*-like sp. (GenBank accession no. AY958888); Eg, *Eggerthella* sp.; Gv, G. *vaginalis;* Pr, uncultured *Prevotella* sp. (GenBank accession no. AY959212); Lc, *Lactobacillus crispatus* group. The range of values of the ordinate of T-RFLP patterns was 0-800 RFU (a) and 0-2040 RFU (b). Note that some minor peaks could not be assigned to a bacterial taxon.

**References**

Coolen et al. Characterization of the microbial flora of the human vulva by analysis of terminal restriction fragment polymorphism (T-RFLP) of 16s rDNA genes. Abstracts of the General Meeting of the American Society for Microbiology. vol. 101, p. 262 (2001).

Moore et al. Human fecal Flora: The Normal Flora of 20 Japanese-Hawaiians Applied Microbiology vol. 27, pp. 961-979 (1974).

Moore et al. 95 Intestinal Floras of Populations That Have a High Risk of Colon Cancer Applied and Environmental Microbiology vol. 61 pp. 3202-3207 (1995).

Abdo et al., "Statistical methods for characterizing diversity of microbial communities by analysis of terminal restriction fragment length polymorphisms of 16S rRNA genes," Environ. Microbiol., 8(5):929-938, 2006.

Antonio et al., "The Identification of Vaginal Lactobacillus Species and the Demographic and Microbiologic Characteristics of Women Colonized by These Species," J. Infect. Dis., 180:1950-1956, 1999.

Bergeron et al., "New DNA-based PCR approaches for rapid real-time detection and prevention of group B streptococcal infections in newborns and pregnant women," Exp. Rev. Mol. Med., (01)00380-5a.pdf, Nov. 8, 2001.

Burton et al., "Evaluation of the Bacterial Vaginal Flora of 20 Postmenopausal Women by Direct (Nugent Score) and Molecular (Polymerase Chain Reaction and Denaturing Gradient Gel Electrophoresis) Techniques," J. Infect. Dis., 186:1770-1780, 2002.

Burton et al., "Improved Understanding of the Bacterial Vaginal Microbiota of Women before and after Probiotic Instillation," Appl. Enviro. Microbiol., 69(1):97-101, 2003.

Burton et al., "Detection of Atopobium vaginae in Postmenopausal Women by Cultivation-Independent Methods Warrants Further Investigation," J. Clin. Microbiol., 42(4):1829-1831, 2004.

Cadieux et aL, "Lactobacillus strains and vaginal ecology," JAMA, 287(15):1940-1941, 2002.

Chang et al., "Inhibition of HIV infectivity by a natural human isolate of Lactobacillus jensenii engineered to express functional two-domain CD4," PNAS. 100(20):11672-11677, 2003.

Cole et al., "The Ribosomal Database Project (RDP-II): previewing a new autoaligner that allows regular updates and the new prokaryotic taxonomy," Nucl. Acids Res., 31(1):442-443, 2003.

Cook et al., "Clinical, Microbiological, and Biochemical Factors in Recurrent Bacterial Vaginosis," J. Clin. Microbiol., 30(4):870-877, 1992.

Coolen et al., "Characterization of Microbial Communities Found in the Human Vagina by Analysis of Terminal Restriction Fragment Length Polymorhisms of 16S rRNA Genes," Appl. Env. Microbiol., 71(12):8729-8737, 2005.

Delaney et al., "Nugent Score Related to Vaginal Culture in Pregnant Women," The Am. College of Obstet. And Gynecol., 98(1):79-84, 2001.

Engebretson et al., "Fidelity of Select Restriction Endonucleases in Determining Microbial Diversity by Terminal-Restriction Fragment Length Polymorphism," Appl. Environ. Microbiology, 69(8):4823-4829, 2003.

Eschenbach et al., "Prevalence of Hydrogen Peroxide-Producing Lactobacillus Species in Normal Women and Women with Bacterial Vaginosis," J. Clin. Microbiol., 27(2):251-256, 1989.

Eschenbach et al., "Influence of the Normal Menstrual Cycle on Vaginal Tissue, Discharge, and Microflora ," Clin. Infect. Dis., 30:901-907, 2000.

Ezaki et al., "Proposal of the genera Anaerococcus gen. nov., Peptoniphilus gen. nov. And Gallicola gen. nov. For members of the genus Peptostreptococcus," Int. J. Syst. Evol. Microbiol., 51:1521-1528, 2001.

Falsen et al., "Phenotypic and phylogenetic characterization of a novel Lactobacillus species from human source: description of Lactobacillus iners sp. nov.," Int. J. Syst. Bacteriol., 49:217-221, 1999.

Favier et al., "Molecular Monitoring of Succession of Bacterial Communities in Human Neonates," App. Environ. Microbiol., 68(1):219-226, 2002.

Ferris et al., "Use of Species-Directed 16S rRNA Gene PCR Primers for Detection of Atopobium vaginae in Patients with Bacterial Vaginosis," J. Clin. Microbiol., 42(12):5892-5894, 2004.

Forney et al., "Molecular microbial ecology: land of the one-eyed king," Curr. Opin. Microbiology, 7:210-220, 2004.
Forney et al., "The vaginal flora of healthy women is not always dominated by lactobacillus species," J. Infec. Dis., 194(10):1468-9,2006.

Frank et al., "Culture-Independent Molecular Analysis of Microbial Constituents of the Healthy Human Outer Ear," J. Clin. Microbiol., 41(1):295-303, 2003.

Fredricks, D. N. & Marrazzo, J. M. (2005). Molecular methodology in determining vaginal flora in health and disease:

its time has come. Curr Infect Dis Rep 7, 463-470.

Gupta et al., "Inverse association of H2O2-producing Lactobacilli and vaginal Escherichia coli colonization in women with recurrent urinary tract infections," J. Infect. Dis., 178:446-450, 1998.

Gupta et al. "Effects of Contraceptive Method on the Vaginal Microbial Flora: A Prospective Evaluation," J. Infect. Dis., 181:595-601, 2000.

Hay, P.E., "Bacterial vaginosis and miscarriage," Curr. Opin. Infect. Dis., 17:41-44, 2004.

Hilton et al., "Lactobacillus GG Vaginal Suppositories and Vaginitis," J. Clin. Microbiol., 33(5):1433, 1995.

Ison et al., "Validation of a simplified grading of Gram stained vaginal smears for use in genitourinary medicine clinics," Sex. Transm. Infect., 78:413-415, 2002.

Jovita et al., "Characterization of a Novel Atopobium Isolate from the Human Vagina: Description of Atopobium vaginae sp. nov.," Int. J. Syst. Bacteriol., 49:1573-1576, 1999.

Hyman, R. W., Fukushima, M., Diamond, L., Kumm, J., Giudice, L. C. & Davis, R. W. (2005). Microbes on the human vaginal epithelium. Proc Natl Acad Sci U S A 102, 7952-7957.

Jukes et al., "Evolution of Protein Molecules," Mammalian Protein Metabolism, Academic Press, Inc., New York and London, 1969.

Kazor et al., "Diversity of Bacterial Populations on the Tongue Dorsa of Patients with Halitosis and Healthy Patients," J. Clin. Micrbiol., 41(2):558-563, 2003.

Kitts, C.L., "Terminal restriction fragment patterns: a tool for comparing microbial communities and assessing community dynamics," Curr. Issues Infest. Microbiol., 2(1):17-25, 2001.

Klebanoff et al., "Viricidal Effect of Lactobacillus acidophilus on Human Immunodeficiency Virus Type 1: Possible Role in Heterosexual Transmission," J. Exp. Med., 174:289-292, 1991.

Kroes et al., "Bacterial diversity within the human subgingival crevice," PNAS, 96(25):14547-14552, 1999.

Larsen et al., "Understanding the Bacterial Flora of the Female Genital Tract," Clin. Infect. Dis., 32:e69-e77, 2001.

Lindner et al., "Quantitative studies of the vaginal flora of healthy women and of obstetric and gynaecological patients," J. Med. Microbiol., 11:233-241, 1978.

Liu et al., "Characterization of Microbial Diversity by Determining Terminal Restriction Fragment Length Polymorphisms of Genes Encoding 16S rRNA," Appl. Env. Microbiol., 63(11):4516-4522, 1997.

Martin et al., "Vaginal Lactobacilli, Microbial Flora, and Risk of Human Immunodeficiency Virus Type 1 and Sexually Transmitted Disease Acquisition," J. Infect. Dis., 180:1863-1868, 1999.

Masfari et al., "Quantitative studies of vaginal bacteria," Genitourin Med., 62:256-263, 1986.

Massi et al., "Identification method based on PCR combined with automated ribotyping for tracking probiotic Lactobacillus strains colonizing the human gut and vagina," J. Appl. Microbiol., 96:777-786, 2004.

McClean et al., "Characterization and selection of a Lactobacillus species to re-colonize the vagina of women with recurrent bacterial vaginosis," J. Med. Microbiol. 49:543-552, 2000.

Milligan et at, "An Examination of Procedures for Determining the Number of Clusters in a Data Set," Psychometrika, 50(2):159-179, 1985.

Newton et al., "Predictors of the vaginal microflora," Am. J. Obstet. Gynecol., 184(5):845-855, 2001.

Nugent et al., "Reliability of Diagnosing Bacterial Vaginosis is Improved by a Standardized Method of Gram Stain Interpretation," J. Clin. Microbiol., 29:297-301, 1991.

O'Dowd et al., "Evaluation of a rapid diagnostic test for bacterial vaginosis," Br. J. Obstet. Gynaecol., 103:366-370, 1996. Oda et al., "Genotypic and Phenotypic Diversity within Species of Purple Nonsulfur Bacteria Isolated from Aquatic Sediments," Appl. Env. Microbiol., 68(7)3467-3477, 2002.

Oda et al., "Biogeography of the Purple NonSulfur Bacterium Rhodopseudomonas palustris," Appl. Env. Microbiol., 69(9):5186-5191, 2003.

Osborn et al., "An evaluation of terminal-restriction fragment length polymorphism (T-RFLP) analysis for the study of microbial community structure and dynamics," Environ. Microbiol., 2(1):39-50, 2002.

Paster et al., "Bacterial Diversity in Human Subgingival Plaque," J. Bacteriol., 183(12):3770-3783, 2001.

Pei et al., "Bacterial biota in the human distal esophagus," PNAS, 101(12):4250-4255, 2004.

Pereira, L., Culhane, J., McCollum, K., Agnew, K. & Nyirjesy, P. (2005). Variation in microbiologic profiles among pregnant women with bacterial vaginosis. Am J Obstet Gynecol 193, 746-751.

Pybus et al., "Microbial interactions in the vaginal ecosystem, with emphasis on the pathogenesis of bacterial vaginosis," Microbes Infect., 1:285-292, 1999.

Redondo-Lopez et al., "Emerging Role of Lactobacilli in the Control and Maintenance of the Vaginal Bacterial Microflora," Rev. Infect. Dis., 12(5):856-872, 1990.

Reid et at., "Identification and plasmid profiles of Lactobacillus species from the vagina of 100 healthy women," FEMS. Immunol. Med. Microbiol., 15:23-26, 1996.

Saitou et al., "The Neighbor-joining Method: A New Method for Reconstructing Phylogenetic Trees," Mol. Biol. Evol., 4(4):406-425, 1987.

Schwebke, J.R., "Bacterial Vaginosis," Curr. Infect. Dis. Rep., 2:14-17, 2000.

Shukla, S. K., Meier, P. R., Mitchell, P. D., Frank, D. N. & Reed, K. D. (2002). Leptotrichia amnionii sp. nov., a novel bacterium isolated from the amniotic fluid of a woman after intrauterine fetal demise. J Clin Microbiol 40, 3346-3349.

Sobel, J.D., "Bacterial Vaginosis," Annu. Rev. Med., 51:349-356, 2000.

Tärnberg et al. "Identification of randomly selected colonies of lactobacilli from normal vaginal fluid by pyrosequencing of the 16S rDNA variable V1 and V3 regions,"APMIS, 110:802-810, 2002.

Thompson et al., "Clustal W: improving the sensitivity of progressive multiple sequence alignment through sequence weighting, position-specific gap penalties and weight matrix choice," Nucleic Acids Research, 22(22):4673-4680, 1994. Vasquez et al., "Vaginal Lactobacillus Flora of Healthy Swedish Women," J. Clin. Microbiol., 40(8):2746-2749, 2002. Ventura et al., "Analysis, Characterization, and Loci of the tuf Genes in Lactobacillus and Bifidobacterium Species and Their Direct Application for Species Identification," Appl. Environ. Microbiol., 69(11):6908-6922, 2003.

Verhelst et al., "Cloning of 16S rRNA genes amplified from normal and disturbed vaginal microflora suggests a strong association between Atopobium vaginae, Gardnerella vaginalis and bacterial vaginosis," BMC Microbiology, 4:11 pages, Apr. 21, 2004.

Verhelst, R., Verstraelen, H., Claeys, G., Verschraegen, G., Van Simaey, L., De Ganck, C., De Backer, E., Temmerman, M. & Vaneechoutte. M. (2005). Comparison between Gram stain and culture for the characterization of vaginal microbiota: definition of a distinct grade that resembles grade I microbiota and revised categorization of grade I microbiota. BMC Microbiol 5, 61-71

Wilks et al, "Quantitative bacteriology of the vaginal flora during the menstrual cycle," J. Med. Microbiol., 24:241-245, 1987.

Wolrath et al., "Analysis of Bacterial Vaginosis-Related Amines in Vaginal Fluid by Gas Chromatography and Mass Spectrometry," J. Clin. Microbiol., 39(11):4026-4031, 2001.

Yu et al., "Estimation of sample size and assurance probability in library screening," Biotechniques, 10(6):776-777, 1991. Zhong et al., "Differentiation of Lactobacillus Species by Molecular Typing," Appl. Environ. Microbiology, 64(7):2418-2423,1998.

Zhou et al., "Characterization of vaginal microbial communities in adult healthy women using cultivation-independent methods," Microbiology, 150:2565-2573, 2004.

Zoetendal et al., "Molecular ecological analysis of the gastrointestinal microbiota: a review," J. Nutr., 134(2):465-472, 2004. Amsel et al., "Nonspecific vaginitis. Diagnostic criteria and microbial and epidemiological associations," Am. J. Med., 74:14-22, 1983.

Bartlett et al., Quantitative bacteriology of the vaginal flora, J. Infect. Dis., 136:271-277, 1977. Francesca et al., "Quantitative and qualitative determination of the vaginal microbial flora by real time PCR," Microbicides, 2004 (abstract only).

Hawes et al., "Hydrogen peroxide-producing Lactobacilli and acquisition of vaginal infections," J. Infect. Dis., 174:1058-1063, 1996.

Hughes and Hillier, "Microbiologic characteristics of Lactobacillus products used for colonization of the vagina," Obstet. Gynecol., 75:244-248, 1990.

Jovita et al., "Characterization of a novel Atopobium isolate from the human vagina: description of Atopobium vaginae sp. nov.," Int. J. Syst. Bacteriol., 49:1573-1576, 1999.

Marrazzo, J., "Bacterial vaginosis," Curr. Treat. Opt. Infect. Dis. 5:63-68, 2003.

Schmid, G.P., "The epidemiology of bacterial vaginosis," Int. J. Gynaecol. Obstet., 67:517-820, 1999.

Schwebke, J.R., "Diagnostic method for bacterial vaginosis," Int. J. Gynaecol. Obstet., 67:521-523, 1999.

Silvester and Dicks, "Identification of lactic acid bacteria isolated from human vaginal secretions," Antonie van Leeuwenhoek, 83:117-123, 2003.

Verhelst, R., Verstraelen, H., Claeys, G., Verschraegen, G., Delanghe, J., Van Simaey, L., De Ganck, C., Temmerman, M. & Vaneechoutte, M. (2004). Cloning of 16S rRNA genes amplified from normal and disturbed vaginal microbiota suggests a strong association between Atopobium vaginae, Gardnerella vaginalis and bacterial vaginosis. BMC Microbiol 4, 16-25.

Verhelst, R., Verstraelen, H., Claeys, G., Verschraegen, G., Van Simaey, L., De Ganck, C., De Backer, E., Temmerman, M. & Vaneechoutte, M. (2005). Comparison between Gram stain and culture for the characterization of vaginal microbiota: definition of a distinct grade that resembles grade I microbiota and revised categorization of grade I microbiota. BMC Microbiol 5, 61-71.

Zhou, X., Bent, S. J., Schneider, M. G., Davis, C. C., Islam, M. R. & Forney, L. J. (2004). Characterization of vaginal microbial communities in adult healthy women using cultivation-independent methods. Microbiology 150, 2565-2573.

## Claims

1. A method for the qualitative detection of microbes in a sample comprising:

   a) of microbes under conditions that give access to their nucleic acid;
   b) amplification of at least a portion of the microbial nucleic acid with at least one primer pair consisting of one labelled and one unlabeled oligonucleotide or of two differently labelled oligonucleotides;

c) sequence-specific fragmentation of the amplified DNA;

d) determination of the length of the DNA fragments into which the labelled oligonucleotide is incorporated;

e) comparison of the length of the DNA fragments of step (c) with DNA fragments predicted from the DNA sequence of microbes, wherein

    i. the nucleotide sequence is taken from a database and
    ii. the DNA sequence is fragmented according to the principles underlying the sequence specific fragmentation of step (c); and

f) detecting whether a microbe is present in said sample whereby the Identity in length

    i. of the DNA fragment referred to in step (d) and
    ii. of a DNA fragment predicted from the DNA sequence of the-microbe referred to in step (e) is indicative of the presence of said microbe in said sample.

2. A method for the quantitative detection of microbes in a sample comprising:

    a. lysis of microbes under conditions that give access to their nucleic acid;
    b. amplification of at least a portion of the microbial nucleic acid with at least one primer pair consisting of one labeled and one unlabeled oligonucleotide or of two differently labelled oligonucleotides;
    c. sequence-specific fragmentation of the amplified DNA;
    d. determination of the length of the DNA fragments into which the labelled oligonucleotide is incorporated;
    e. comparison of the length of the DNA fragments of step (c) with DNA fragments predicted from the nucleotide sequence of microbes, wherein

        i. the nucleotide sequence is chosen from a database and
        ii. the DNA sequence is fragmented according to the principles underlying the sequence-specific fragmentation of step (c);

    f. detecting whether a microbe is present in said sample, whereby the Identity in length

        i. of the DNA fragment referred to in step (d) and
        ii. of a DNA fragment predicted from the DNA sequence of the microbe referred to in step (e) is indicative of the presence of said microbe in said sample; and

    g. quantification of microbes by

        i. comparing the specific signal of DNA amplified from the microbe to an internal nucleic acid quantity standard;
        ii. calculating the absolute amount of amplified DNA; and
        iii. calculating the quantity of microbes present in the sample.

3. The method of claim 2, wherein the internal nucleic acid quantity standard is added prior to lysis, comprising:

    a. the internal DNA quantity standard is homologous to an amplified microbial DNA, wherein the DNA comprises repetitive sequences and/or is selected from the group consisting of 16S rDNA, 23S rDNA, TUF, HSPs, 1TS, or DNA sequences of similar quality..
    b. the internal DNA quantity standard is generated by adding a defined quantity of a microbe to the sample prior to lysis.
    c. The size standard of claim 6, wherein the size standard is labelled.
    d. The labelled size standard of claim 7, wherein the label differs from the label of the oligonucleotides used for amplification of the DNA of the microbe.
    e. the size standard is a mixture of DNA fragments in the range of 50 to 800 bases.

4. The method of any one of claims 1 to 3, wherein the amplified DNA and/or the nucleic acid of the microbe in the sample is isolated and/or concentrated.

5. The method of any one of claims 1 to 4, wherein the nucleic acid of the microbe is amplified by RT-PCR, PCR, LCR, CPR, SDA, LAMP, NASBA,RCA, 3SR, QBR, bDNA.

6. The method of any one of claims 1 to 5, wherein the DNA to be amplified comprises at least one variable domain flanked by at least two conserved domains.

7. The method of claim 6, wherein the oligonucleotides used for DNA amplification hybridize with the conserved sequences.

8. The method of any one of claims 1 to 7, wherein the amplified nucleic acid contains nucleotides homologous to a nucleotide sequence selected from the group consisting of 16S rDNA, 23S rDNA, TUF, HSPs, ITS and any other suitable gene group.

9. The method of any one of claims 1 to 8, wherein the specific fragmentation is performed with at least one restriction enzyme.

10. The method of any one of claims 1 to 9, wherein the DNA fragment length is determined by DNA electrophoresis, HPLC, chromatography, mass spectrometry or oligonucleotide hybridization.

11. The method of claim 10, wherein DNA electrophoresis is performed on:

    a. a capillary sequencer;
    b. a multi-capillary sequencer; or
    c. a high resolution polyacrylamide gel sequencer
    d. A microfluid-chip-based capillary electrophoresis system.

12. The method of any one of claims 1 to 11, wherein more than one pair of oligonucleotides is used for nucleic acid amplification.

13. The method of any one of claims 1 to 12, wherein at least one DNA sequence is amplified from one or more microbes.

14. The method of any one of claims 1 to 13, wherein the microbe is selected from the group consisting of bacteria, protozoa, fungi, viruses and/or parasites including all known kingdoms and phyla.

15. The method of claims 13 or 14, wherein the detection is species specific.

16. The method of any one of claims 1 to 15, wherein the sample represents a sample selected from the group consisting of water, dust, soil, sputum, urine, blood, plant, and samples from any human or animal location.

17. The method of any one of claims 1 to 16, wherein the methods define one or more normal, disturbed and/or vaginosis-associated vaginal microbial communities in pregnant and non-pregnant women, the method comprising: obtaining a plurality of single vaginal samples, wherein each single sample comprises vaginal microorganisms obtained from a female with or without vaginal pathology; using analytical laboratory techniques to obtain a plurality of microbial profiles, wherein each microbial profile is obtained from the single sample of vaginal microorganisms obtained from a female with or without vaginal pathology; identifying a plurality of consensus profiles from among the plurality of microbial profiles, thereby defining a plurality of normal, disturbed, and/or vaginosis-associated vaginal microbial communities, wherein the plurality of consensus profiles comprise the plurality of consensus profiles to a user.

18. The method of any one of claims 1 to 17, wherein the method makes use of a databank storing the nucleic acid sequence information of the microbes referred to in claim 13 or 14 and a computer program enabled to execute the steps of:

    a. searching in the data bank for sequences homologous to the oligonucleotides used for the nucleic acid amplification and copying homologous nucleotide sequences into a file;
    b. searching for the presence of restriction enzyme recognition sequences within the DNA sequences stored in the data bank generated in step (a); and
    c. performing an *in silico* restriction digest of said DNA by using the restriction enzyme recognition sequences of step (b);

d. performing an *in silico* restriction digest of said DNA and/or RNA sequences stored in data bases from deep sequencing studies.

19. A kit for the qualitative and/or quantitative detection of a microbe or for the simultaneous qualitative and quantitative detection of several different microbes or for targeting the analysis of structural diversity of different communities (such as bacterial, fungal, archael, viral, etc.) comprising:

   a. at least one primer pair, consisting of a labeled and an unlabeled oligonucleotide or two or more differently labeled oligonucleotides, to allow the amplification of the nucleic acid of the microbe referred to in claim 13 or 14;
   b. at least one nucleic acid or DNA quantity standard as referred to in any one of claims 3 to 5; in one or more containers and/or
   c. the computer program as referred to in claim 18.

20. A kit for the specifically characterization of a normal, disturbed and pathological microflora in vaginal samples from non-pregnant and pregnant women, comprising:

   a. at least one primer pair, consisting of a labeled and an unlabeled oligonucleotide or two or more differently labeled oligonucleotides, to allow the amplification of the nucleic acid of the microbe referred to in claim 13 or 14;
   b. at least one nucleic acid or DNA quantity standard as referred to in any one of claims 3 to 5; in one or more containers and/or
   c. the computer program as referred to in claim 18.

**Figure 1**

Terminal restriction fragment length (bp)

**Europäisches Patentamt**

**European Patent Office**

**Office européen des brevets**

# EUROPEAN SEARCH REPORT

Application Number

EP 15 00 0655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | F. L. THIES ET AL: "Rapid characterization of the normal and disturbed vaginal microbiota by application of 16S rRNA gene terminal RFLP fingerprinting", JOURNAL OF MEDICAL MICROBIOLOGY., vol. 56, no. 6, 1 June 2007 (2007-06-01), pages 755-761, XP55223499, GB ISSN: 0022-2615, DOI: 10.1099/jmm.0.46562-0 * the whole document * | 1-20 | INV. C12Q1/68 |
| X | US 2006/172330 A1 (OSBORN THOMAS [US] ET AL) 3 August 2006 (2006-08-03) * see whole doc. esp. claims and pars, 27, 209 * | 1-20 | |
| X | SCHMIDT JULIA K ET AL: "Characterization of a three bacteria mixed culture in a chemostat: Evaluation and application of a quantitative terminal-restriction fragment length polymorphism (T-RFLP) analysis for absolute and species specific cell enumeration", BIOTECHNOLOGY AND BIOENGINEERING, vol. 96, no. 4, March 2007 (2007-03), pages 738-756, XP002748773, ISSN: 0006-3592 * the whole document * | 1-20 | TECHNICAL FIELDS SEARCHED (IPC) C12Q |

-/--

The present search report has been drawn up for all claims

| Place of search | Date of completion of the search | Examiner |
|---|---|---|
| Munich | 27 October 2015 | Mueller, Frank |

Europäisches
Patentamt

European
Patent Office

Office européen
des brevets

# EUROPEAN SEARCH REPORT

Application Number

EP 15 00 0655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | M. J. L. COOLEN ET AL: "Characterization of Microbial Communities Found in the Human Vagina by Analysis of Terminal Restriction Fragment Length Polymorphisms of 16S rRNA Genes", APPLIED AND ENVIRONMENTAL MICROBIOLOGY, vol. 71, no. 12, 1 December 2005 (2005-12-01), pages 8729-8737, XP55223528, US ISSN: 0099-2240, DOI: 10.1128/AEM.71.12.8729-8737.2005 * the whole document * | 1-20 | |
| A | WO 2014/005094 A1 (TAXON BIOSCIENCES INC [US]) 3 January 2014 (2014-01-03) * the whole document * | 1-20 | |

TECHNICAL FIELDS
SEARCHED        (IPC)

The present search report has been drawn up for all claims

| Place of search | Date of completion of the search | Examiner |
|---|---|---|
| Munich | 27 October 2015 | Mueller, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 00 0655

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2015

| Patent document cited in search report | | | Publication date | Patent family member(s) | | | Publication date |
|---|---|---|---|---|---|---|---|
| US 2006172330 | A1 | | 03-08-2006 | US | 2006172330 | A1 | 03-08-2006 |
| | | | | US | 2010226899 | A1 | 09-09-2010 |
| | | | | US | 2011053802 | A1 | 03-03-2011 |
| WO 2014005094 | A1 | | 03-01-2014 | CA | 2840459 | A1 | 03-01-2014 |
| | | | | EP | 2694669 | A1 | 12-02-2014 |
| | | | | US | 2014162274 | A1 | 12-06-2014 |
| | | | | WO | 2014005094 | A1 | 03-01-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 13173893 A
- EP 320308 A
- WO 8810315 A, Gingeras
- US 74612191 A

### Non-patent literature cited in the description

- **HOLMES et al.** *Sexually Transmitted Diseases,* 1999
- **MARTIN et al.** *J Infect Dis,* 1998, vol. 178, 1053-1059
- **SCHMID et al.** *Sex Transm Infect,* 2000, vol. 76, 3-4
- **TAHA et al.** *AIDS,* 1998, vol. 12, 1699-1706
- **SOBEL.** *Annu Rev Med,* 2000, vol. 51, 349-356
- **SCHWEBKE.** *Curr Infect Dis Rep,* 2000, vol. 2, 14-17
- **GUPTA et al.** *J Infect Dis,* 1998, vol. 178, 446-450
- **HAWES et al.** *J Infect Dis,* 1996, vol. 174, 1058-1063
- **PYBUS ; ONDERDONK.** *Microbes Infect,* 1999, vol. 1, 285-292
- **LEITICH et al.** *Am J Obstet Gynecol,* 2003, vol. 189, 139-147
- **RALPH et al.** *BMJ,* 1999, vol. 319, 220-223
- **SWEET.** *Infect Dis Obstet Gynecol,* 2000, vol. 8, 184-190
- **HILLIER et al.** *N Engl J Med,* 1995, vol. 333, 1737-1742
- **AMANN et al.** *Microbiol. Rev.,* 1995, vol. 59, 143-169
- **BARNS.** *Nati. Acad. Sci. USA,* 1994, vol. 91, 1609-1613
- **GIOVANNONI et al.** *Nature,* 1990, vol. 345, 60-63
- **TORSVIK.** *Appl. Environ. Microbiol.,* 1990, vol. 56, 782-787
- **SWEET.** *Infect. Dis. Obstet. Gynecol.,* 2000, vol. 8, 184-190
- **SCHMID.** *Int. J. Gynaecol. Obstet.,* 1999, vol. 67 (1), S17-S20
- **SCHWEBKE.** *Int. J. Gynaecol. Obstet.,* 1999, vol. 67 (1), S21-S23
- **ANTONIO et al.** *J. Infect. Dis.,* 1999, vol. 180, 1950-1956
- **PAVLOVA et al.** *J. Appl. Microbiol.,* 2002, vol. 92, 451-459
- **REID et al.** *FEMS. Immunol. Med. Microbiol.,* 1996, vol. 15, 23-26
- **ESCHENBACH et al.** *Clin. Infect. Dis.,* 2000, vol. 30, 901-907
- **HILLIER.** *AIDS Res. Hum. Retroviruses,* 1998, vol. 14 (1), S17-S21
- **LARSEN MONIF.** *Clin. Infect. Dis.,* 2001, vol. 32, e69-e77
- **MARRAZZO et al.** *J. Infect. Dis.,* 2002, vol. 185, 1307-1313
- **REDONDO-LOPEZ et al.** *Rev. Infect. Dis.,* 1990, vol. 12, 856-872
- **KROES et al.** *Microbiol.,* 1999, vol. 96, 14547-14552
- **PASTER et al.** *J. Bacteriol.,* 2001, vol. 183, 3770-3783
- **FAVIER et al.** *App. Environ. Microbiol.,* 2002, vol. 68, 219-226
- **ZOETENDAL et al.** *J. Nutr.,* 2004, vol. 134, 465-472
- **FRANK et al.** *J. Clin. Microbiol.,* 2003, vol. 41, 295-303
- **KAZOR et al.** *J. Clin. Microbiol.,* 2003, vol. 41, 558-563
- **PEI et al.** *Proc. Natl. Acad. Sci. USA,* 2004, vol. 101, 4250-4255
- **LIU et al.** *Appl. Environ, Microbiol.,* 1997, vol. 63, 4516-4522
- **BENJAMIN LEWIN.** Genes V. Oxford University Press, 1994
- The Encyclopedia of Molecular Biology. Blackwell Science Ltd, 1994
- Molecular Biology and Biotechnology: a Comprehensive Desk Reference. VCH Publishers, Inc, 1995
- **ROTBART et al.** PCR Technology. Stockton Press, 1989
- **HAN.** *Biochemistry,* 1987, vol. 26, 1617-1625
- **WALKER et al.** *Nucleic Acid Res.,* 1992, 1691-1696
- **KWÖH et al.** *Proc. Nat. Acad. Sci. USA,* 1989, vol. 86, 1173

- **ERLICH, H, A.** PCR Technology. Stockton Press, 1989
- **INNIS, M. A. ; D. H. GELFAND ; J. J. SNINSKY ; T. J. WHITE.** PCR Protocols: A guide to methods and apprications. Academic Press, 1990
- **MAIFLAK BL ; COLE JR ; LILBURN TG ; PARKER CT ; JR, SAXMAN PR ; FARRIS RJ ; GARRITY GM ; OLSEN GJ ; SCHMIDT TM ; TIEDJE JM.** The RDP-11 (Ribosomal Database Project). *Nucleic Acids Res,* 01 January 2001, vol. 29 (1), 173-4
- *Advances in Applied Mathematics,* 1981, vol. 2, 482-489
- **BRUTLAG.** *Camp. App. Biosci.,* 1990, vol. 6, 237-245
- **MANIATIS et al.** Molecular Cloning: A laboratory manual. Cord Spring Harbor Press, 1982
- **S. JANE FLINT.** Principles of Virology: Molecular Biology, Pathogenesis, and Control. Amer Society for Microbiology
- **MANIATIS et al.** Molecular Cloning: A laboratory manual. Cold Spring Harbor Press, 1982
- **PERBAL.** A Practica] Guide to Molecular Cloning. Wiley & Sons, 1984
- **COOLEN et al.** Characterization of the microbial flora of the human vulva by analysis of terminal restriction fragment polymorphism (T-RFLP) of 16s rDNA genes. *Abstracts of the General Meeting of the American Society for Microbiology,* 2001, vol. 101, 262
- **MOORE et al.** *Human fecal Flora: The Normal Flora of 20 Japanese-Hawaiians Applied Microbiology,* 1974, vol. 27, 961-979
- **MOORE et al.** *95 Intestinal Floras of Populations That Have a High Risk of Colon Cancer Applied and Environmental Microbiology,* 1995, vol. 61, 3202-3207
- **ABDO et al.** Statistical methods for characterizing diversity of microbial communities by analysis of terminal restriction fragment length polymorphisms of 16S rRNA genes. *Environ. Microbiol.,* 2006, vol. 8 (5), 929-938
- **ANTONIO et al.** The Identification of Vaginal Lactobacillus Species and the Demographic and Microbiologic Characteristics of Women Colonized by These Species. *J. Infect. Dis.,* 1999, vol. 180, 1950-1956
- **BERGERON et al.** New DNA-based PCR approaches for rapid real-time detection and prevention of group B streptococcal infections in newborns and pregnant women. *Exp. Rev. Mol. Med.,* 08 November 2001
- **BURTON et al.** Evaluation of the Bacterial Vaginal Flora of 20 Postmenopausal Women by Direct (Nugent Score) and Molecular (Polymerase Chain Reaction and Denaturing Gradient Gel Electrophoresis) Techniques. *J. Infect. Dis.,* 2002, vol. 186, 1770-1780
- **BURTON et al.** Improved Understanding of the Bacterial Vaginal Microbiota of Women before and after Probiotic Instillation. *Appl. Enviro. Microbiol.,* 2003, vol. 69 (1), 97-101
- **BURTON et al.** Detection of Atopobium vaginae in Postmenopausal Women by Cultivation-Independent Methods Warrants Further Investigation. *J. Clin. Microbiol.,* 2004, vol. 42 (4), 1829-1831
- **CADIEUX et al.** Lactobacillus strains and vaginal ecology. *JAMA,* 2002, vol. 287 (15), 1940-1941
- **CHANG et al.** Inhibition of HIV infectivity by a natural human isolate of Lactobacillus jensenii engineered to express functional two-domain CD4. *PNAS,* 2003, vol. 100 (20), 11672-11677
- **COLE et al.** The Ribosomal Database Project (RDP-II): previewing a new autoaligner that allows regular updates and the new prokaryotic taxonomy. *Nucl. Acids Res.,* 2003, vol. 31 (1), 442-443
- **COOK et al.** Clinical, Microbiological, and Biochemical Factors in Recurrent Bacterial Vaginosis. *J. Clin. Microbiol.,* 1992, vol. 30 (4), 870-877
- **COOLEN et al.** Characterization of Microbial Communities Found in the Human Vagina by Analysis of Terminal Restriction Fragment Length Polymorhisms of 16S rRNA Genes. *Appl. Env. Microbiol.,* 2005, vol. 71 (12), 8729-8737
- **DELANEY et al.** Nugent Score Related to Vaginal Culture in Pregnant Women. *The Am. College of Obstet. And Gynecol.,* 2001, vol. 98 (1), 79-84
- **ENGEBRETSON et al.** Fidelity of Select Restriction Endonucleases in Determining Microbial Diversity by Terminal-Restriction Fragment Length Polymorphism. *Appl. Environ. Microbiology,* 2003, vol. 69 (8), 4823-4829
- **ESCHENBACH et al.** Prevalence of Hydrogen Peroxide-Producing Lactobacillus Species in Normal Women and Women with Bacterial Vaginosis. *J. Clin. Microbiol.,* 1989, vol. 27 (2), 251-256
- **ESCHENBACH et al.** Influence of the Normal Menstrual Cycle on Vaginal Tissue, Discharge, and Microflora. *Clin. Infect. Dis.,* 2000, vol. 30, 901-907
- **EZAKI et al.** Proposal of the genera Anaerococcus gen. nov., Peptoniphilus gen. nov. And Gallicola gen. nov. For members of the genus Peptostreptococcus. *Int. J. Syst. Evol. Microbiol.,* 2001, vol. 51, 1521-1528
- **FALSEN et al.** Phenotypic and phylogenetic characterization of a novel Lactobacillus species from human source: description of Lactobacillus iners sp. nov. *Int. J. Syst. Bacteriol.,* 1999, vol. 49, 217-221
- **FAVIER et al.** Molecular Monitoring of Succession of Bacterial Communities in Human Neonates. *App. Environ. Microbiol.,* 2002, vol. 68 (1), 219-226

- **FERRIS et al.** Use of Species-Directed 16S rRNA Gene PCR Primers for Detection of Atopobium vaginae in Patients with Bacterial Vaginosis. *J. Clin. Microbiol.,* 2004, vol. 42 (12), 5892-5894
- **FORNEY et al.** Molecular microbial ecology: land of the one-eyed king. *Curr. Opin. Microbiology,* 2004, vol. 7, 210-220
- **FORNEY et al.** The vaginal flora of healthy women is not always dominated by lactobacillus species. *J. Infec. Dis.,* 2006, vol. 194 (10), 1468-9
- **FRANK et al.** Culture-Independent Molecular Analysis of Microbial Constituents of the Healthy Human Outer Ear. *J. Clin. Microbiol.,* 2003, vol. 41 (1), 295-303
- **FREDRICKS, D. N. ; MARRAZZO, J. M.** Molecular methodology in determining vaginal flora in health and disease: its time has come. *Curr Infect Dis Rep,* 2005, vol. 7, 463-470
- **GUPTA et al.** Inverse association of H202-producing Lactobacilli and vaginal Escherichia coli colonization in women with recurrent urinary tract infections. *J. Infect. Dis.,* 1998, vol. 178, 446-450
- **GUPTA et al.** Effects of Contraceptive Method on the Vaginal Microbial Flora: A Prospective Evaluation. *J. Infect. Dis.,* 2000, vol. 181, 595-601
- **HAY, P.E.** Bacterial vaginosis and miscarriage. *Curr. Opin. Infect. Dis.,* 2004, vol. 17, 41-44
- **HILTON et al.** Lactobacillus GG Vaginal Suppositories and Vaginitis. *J. Clin. Microbiol.,* 1995, vol. 33 (5), 1433
- **ISON et al.** Validation of a simplified grading of Gram stained vaginal smears for use in genitourinary medicine clinics. *Sex. Transm. Infect.,* 2002, vol. 78, 413-415
- **JOVITA et al.** Characterization of a Novel Atopobium Isolate from the Human Vagina: Description of Atopobium vaginae sp. nov. *Int. J. Syst. Bacteriol.,* 1999, vol. 49, 1573-1576
- **HYMAN, R. W. ; FUKUSHIMA, M. ; DIAMOND, L. ; KUMM, J. ; GIUDICE, L. C. ; DAVIS, R. W.** Microbes on the human vaginal epithelium. *Proc Natl Acad Sci U S A,* 2005, vol. 102, 7952-7957
- Evolution of Protein Molecules. **JUKES et al.** Mammalian Protein Metabolism. Academic Press, Inc, 1969
- **KAZOR et al.** Diversity of Bacterial Populations on the Tongue Dorsa of Patients with Halitosis and Healthy Patients. *J. Clin. Micrbiol.,* 2003, vol. 41 (2), 558-563
- **KITTS, C.L.** Terminal restriction fragment patterns: a tool for comparing microbial communities and assessing community dynamics. *Curr. Issues Infest. Microbiol.,* 2001, vol. 2 (1), 17-25
- **KLEBANOFF et al.** Viricidal Effect of Lactobacillus acidophilus on Human Immunodeficiency Virus Type 1: Possible Role in Heterosexual Transmission. *J. Exp. Med.,* 1991, vol. 174, 289-292
- **KROES et al.** Bacterial diversity within the human subgingival crevice. *PNAS,* 1999, vol. 96 (25), 14547-14552
- **LARSEN et al.** Understanding the Bacterial Flora of the Female Genital Tract. *Clin. Infect. Dis.,* 2001, vol. 32, e69-e77
- **LINDNER et al.** Quantitative studies of the vaginal flora of healthy women and of obstetric and gynaecological patients. *J. Med. Microbiol.,* 1978, vol. 11, 233-241
- **LIU et al.** Characterization of Microbial Diversity by Determining Terminal Restriction Fragment Length Polymorphisms of Genes Encoding 16S rRNA. *Appl. Env. Microbiol.,* 1997, vol. 63 (11), 4516-4522
- **MARTIN et al.** Vaginal Lactobacilli, Microbial Flora, and Risk of Human Immunodeficiency Virus Type 1 and Sexually Transmitted Disease Acquisition. *J. Infect. Dis.,* 1999, vol. 180, 1863-1868
- **MASFARI et al.** Quantitative studies of vaginal bacteria. *Genitourin Med.,* 1986, vol. 62, 256-263
- **MASSI et al.** Identification method based on PCR combined with automated ribotyping for tracking probiotic Lactobacillus strains colonizing the human gut and vagina. *J. Appl. Microbiol.,* 2004, vol. 96, 777-786
- **MCCLEAN et al.** Characterization and selection of a Lactobacillus species to re-colonize the vagina of women with recurrent bacterial vaginosis. *J. Med. Microbiol.,* 2000, vol. 49, 543-552
- **MILLIGAN.** An Examination of Procedures for Determining the Number of Clusters in a Data Set. *Psychometrika,* 1985, vol. 50 (2), 159-179
- **NEWTON et al.** Predictors of the vaginal microflora. *Am. J. Obstet. Gynecol.,* 2001, vol. 184 (5), 845-855
- **NUGENT et al.** Reliability of Diagnosing Bacterial Vaginosis is Improved by a Standardized Method of Gram Stain Interpretation. *J. Clin. Microbiol.,* 1991, vol. 29, 297-301
- **O'DOWD et al.** Evaluation of a rapid diagnostic test for bacterial vaginosis. *Br. J. Obstet. Gynaecol.,* 1996, vol. 103, 366-370
- **ODA et al.** Genotypic and Phenotypic Diversity within Species of Purple Nonsulfur Bacteria Isolated from Aquatic Sediments. *Appl. Env. Microbiol.,* 2002, vol. 68 (7), 3467-3477
- **ODA et al.** Biogeography of the Purple NonSulfur Bacterium Rhodopseudomonas palustris. *Appl. Env. Microbiol.,* 2003, vol. 69 (9), 5186-5191
- **OSBORN et al.** An evaluation of terminal-restriction fragment length polymorphism (T-RFLP) analysis for the study of microbial community structure and dynamics. *Environ. Microbiol.,* 2002, vol. 2 (1), 39-50
- **PASTER et al.** Bacterial Diversity in Human Subgingival Plaque. *J. Bacteriol.,* 2001, vol. 183 (12), 3770-3783

- **PEI et al.** Bacterial biota in the human distal esophagus. *PNAS,* 2004, vol. 101 (12), 4250-4255
- **PEREIRA, L. ; CULHANE, J. ; MCCOLLUM, K. ; AGNEW, K ; NYIRJESY, P.** Variation in microbiologic profiles among pregnant women with bacterial vaginosis. *Am J Obstet Gynecol,* 2005, vol. 193, 746-751
- **PYBUS et al.** Microbial interactions in the vaginal ecosystem, with emphasis on the pathogenesis of bacterial vaginosis. *Microbes Infect.,* 1999, vol. 1, 285-292
- **REDONDO-LOPEZ et al.** Emerging Role of Lactobacilli in the Control and Maintenance of the Vaginal Bacterial Microflora. *Rev. Infect. Dis.,* 1990, vol. 12 (5), 856-872
- **REID.** Identification and plasmid profiles of Lactobacillus species from the vagina of 100 healthy women. *FEMS. Immunol. Med. Microbiol.,* 1996, vol. 15, 23-26
- **SAITOU et al.** The Neighbor-joining Method: A New Method for Reconstructing Phylogenetic Trees. *Mol. Biol. Evol.,* 1987, vol. 4 (4), 406-425
- **SCHWEBKE, J.R.** Bacterial Vaginosis. *Curr. Infect. Dis. Rep.,* 2000, vol. 2, 14-17
- **SHUKLA, S. K. ; MEIER, P. R. ; MITCHELL, P. D. ; FRANK, D. N. ; REED, K. D.** Leptotrichia amnionii sp. nov., a novel bacterium isolated from the amniotic fluid of a woman after intrauterine fetal demise. *J Clin Microbiol,* 2002, vol. 40, 3346-3349
- **SOBEL, J.D.** Bacterial Vaginosis. *Annu. Rev. Med.,* 2000, vol. 51, 349-356
- **TÄRNBERG et al.** Identification of randomly selected colonies of lactobacilli from normal vaginal fluid by pyrosequencing of the 16S rDNA variable V1 and V3 regions. *APMIS,* 2002, vol. 110, 802-810
- **THOMPSON et al.** Clustal W: improving the sensitivity of progressive multiple sequence alignment through sequence weighting, position-specific gap penalties and weight matrix choice. *Nucleic Acids Research,* 1994, vol. 22 (22), 4673-4680
- **VASQUEZ et al.** Vaginal Lactobacillus Flora of Healthy Swedish Women. *J. Clin. Microbiol.,* 2002, vol. 40 (8), 2746-2749
- **VENTURA et al.** Analysis, Characterization, and Loci of the tuf Genes in Lactobacillus and Bifidobacterium Species and Their Direct Application for Species Identification. *Appl. Environ. Microbiol.,* 2003, vol. 69 (11), 6908-6922
- **VERHELST et al.** Cloning of 16S rRNA genes amplified from normal and disturbed vaginal microflora suggests a strong association between Atopobium vaginae, Gardnerella vaginalis and bacterial vaginosis. *BMC Microbiology,* 21 April 2004, vol. 4, 11
- **VERHELST, R. ; VERSTRAELEN, H. ; CLAEYS, G. ; VERSCHRAEGEN, G. ; VAN SIMAEY, L. ; DE GANCK, C. ; DE BACKER, E. ; TEMMERMAN, M. ; VANEECHOUTTE. M.** Comparison between Gram stain and culture for the characterization of vaginal microbiota: definition of a distinct grade that resembles grade I microbiota and revised categorization of grade I microbiota. *BMC Microbiol,* 2005, vol. 5, 61-71
- **WILKS et al.** Quantitative bacteriology of the vaginal flora during the menstrual cycle. *J. Med. Microbiol.,* 1987, vol. 24, 241-245
- **WOLRATH et al.** Analysis of Bacterial Vaginosis-Related Amines in Vaginal Fluid by Gas Chromatography and Mass Spectrometry. *J. Clin. Microbiol.,* 2001, vol. 39 (11), 4026-4031
- **YU et al.** Estimation of sample size and assurance probability in library screening. *Biotechniques,* 1991, vol. 10 (6), 776-777
- **ZHONG et al.** Differentiation of Lactobacillus Species by Molecular Typing. *Appl. Environ. Microbiology,* 1998, vol. 64 (7), 2418-2423
- **ZHOU et al.** Characterization of vaginal microbial communities in adult healthy women using cultivation-independent methods. *Microbiology,* 2004, vol. 150, 2565-2573
- **ZOETENDAL et al.** Molecular ecological analysis of the gastrointestinal microbiota: a review. *J. Nutr.,* 2004, vol. 134 (2), 465-472
- **AMSEL et al.** Nonspecific vaginitis. Diagnostic criteria and microbial and epidemiological associations. *Am. J. Med.,* 1983, vol. 74, 14-22
- **BARTLETT et al.** Quantitative bacteriology of the vaginal flora. *J. Infect. Dis.,* 1977, vol. 136, 271-277
- **FRANCESCA et al.** Quantitative and qualitative determination of the vaginal microbial flora by real time PCR. *Microbicides,* 2004
- **HAWES et al.** Hydrogen peroxide-producing Lactobacilli and acquisition of vaginal infections. *J. Infect. Dis.,* 1996, vol. 174, 1058-1063
- **HUGHES ; HILLIER.** Microbiologic characteristics of Lactobacillus products used for colonization of the vagina. *Obstet. Gynecol.,* 1990, vol. 75, 244-248
- **JOVITA et al.** Characterization of a novel Atopobium isolate from the human vagina: description of Atopobium vaginae sp. nov. *Int. J. Syst. Bacteriol.,* 1999, vol. 49, 1573-1576
- **MARRAZZO, J.** Bacterial vaginosis. *Curr. Treat. Opt. Infect. Dis.,* 2003, vol. 5, 63-68
- **SCHMID, G.P.** The epidemiology of bacterial vaginosis. *Int. J. Gynaecol. Obstet.,* 1999, vol. 67, 517-820
- **SCHWEBKE, J.R.** Diagnostic method for bacterial vaginosis. *Int. J. Gynaecol. Obstet.,* 1999, vol. 67, 521-523

- **SILVESTER ; DICKS.** Identification of lactic acid bacteria isolated from human vaginal secretions. *Antonie van Leeuwenhoek,* 2003, vol. 83, 117-123
- **VANEECHOUTTE, M.** Cloning of 16S rRNA genes amplified from normal and disturbed vaginal microbiota suggests a strong association between Atopobium vaginae, Gardnerella vaginalis and bacterial vaginosis. *BMC Microbiol,* 2004, vol. 4, 16-25
- **VANEECHOUTTE, M.** Comparison between Gram stain and culture for the characterization of vaginal microbiota: definition of a distinct grade that resembles grade I microbiota and revised categorization of grade I microbiota. *BMC Microbiol,* 2005, vol. 5, 61-71
- **ZHOU, X. ; BENT, S. J. ; SCHNEIDER, M. G. ; DAVIS, C. C. ; ISLAM, M. R. ; FORNEY, L. J.** Characterization of vaginal microbial communities in adult healthy women using cultivation-independent methods. *Microbiology,* 2004, vol. 150, 2565-2573